# EUROPEAN PATENT APPLICATION

(11) **EP 3 139 169 A1**
(43) Date of publication of application: **08.03.2017**
(21) Application number: 15786799.5
(22) Date of filing: 28.04.2015
(51) Int. Cl.: G01N 33/574, A61K 31/445, A61K 39/00, A61P 35/00

(54) **METHOD FOR DIAGNOSING AND MONITORING THE PRESENCE OF CANCER IN A HUMAN SUBJECT**

(30) Priority: 29.04.2014 ES 201430631
(71) Applicant: Servicio Andaluz de Salud, 41071 Sevilla (ES)
(72) Inventor: SALINAS MARTÍN, Manuel Vicente, E-41071 Sevilla (ES)
(74) Representative: Lewis, Graham Matthew
(86) International application number: PCT/ES2015/070352
(87) International publication number: WO 2015/166128

(57) **Abstract**

Method of obtaining useful data for diagnosing the presence of cancer in an individual and to determine the stage or degree of progression of the cancer. Also to determine response to therapy and group subjects into responders and non-responders. Kit or device comprising the elements necessary to carry out this method and its uses.

## Description

### FIELD OF THE INVENTION.

The present invention is within the field of medicine, pharmacy and molecular biology, and more specifically in the field of oncology. Specifically, it relates to a method for diagnosing the presence of cancer, monitoring the progression of a cancer or response to treatment in a human subject as well as the kit or device comprising the necessary elements for carrying out said method and its use.

### STATE OF THE ART.

NK1 receptors (neurokinin 1 receptors or neuropeptide receptors of Substance P and tachykinin), are widely distributed in the body cells. Numerous biological processes in whose regulation involves NK1 receptors are currently known. NK1 receptors are a G protein coupled receptor type.

It has been reported that the NK1 receptor is overexpressed in tumor cells and the use of various antagonists thereof can inhibit cell proliferation (Singh *et al ., 2000;.* Bigioni *et al*, 2005) and more specifically, lung carcinoma cells (Orosz *et al* ., 1995; Bunn *et al,* 1994;.. Muñoz *et al,* 2012), brain tumors (Palma *et al,* 2000), enterochromaffin cells tumors (EP 773 026 -Pfizer- patent), prostatic carcinomas (WO 2001001922), colon and stomach carcinoma (Rosso *et al.,* 2008) or melanoma (Muñoz *et al.,* 2010).

Patent ES 2246687 claims the use of non-peptide NK1 receptor antagonists in the manufacture of a pharmaceutical composition for the production of apoptosis in cancer tumor cells in mammals. The Spanish patent application P201101311 (PCT / ES2012 / 070865) claims the use of modifying agents of the peritumoral microenvironment, in particular antagonists of the NK1 receptors, for the treatment of cancer. Furthermore, the WO2012020162 Patent Application describes the use of antibodies or fragments thereof, against NK1, NK2 and / or NK3 receptors, which is useful in the treatment of cancer. The *TAC1R* gene is the gene which encodes the NK1 receptor. It has been stated that in the cells composing the tissue, the NK1 receptor has two forms: a long (complete) form containing 407 amino acids and a short form (truncated) containing 311 amino acids wherein said receptor lacks its cytoplasmic c-terminus (Kage *et al.,* 1993; Gillespie *et al, 2011*). Both forms are the result of a variant of "splicing" the *TAC1R* gene which encodes the NK1 receptor.

This truncated form of the NK1 receptor is present in cancerous tumor cells (Gillespie *et al.,* 2011). The Spanish patent P201330174 claims a method which includes the detection of this truncated form in tumor tissue to be sued to predict response to treatment of these tumors with NK1 receptor antagonists.

Therefore and in conclusion, the following facts are currently known in the state of the art:
1. The NK1 receptors are widely distributed in the body of the human being.
2. The utilization of non-peptide antagonists of the NK1 receptor can be either useful in the treatment of cancerous tumors by causing death of tumor cells by apoptosis (ES 2246687), modification of the tumor microenvironment (PCT / ES2012 / 070865) or by using antibodies or fragments thereof, against these receptors (WO2012020162).
3. That tumor cells may have a short or truncated form of the NK1 receptor.
4. This truncated form of the NK1 receptor is present in cancerous tumor cells and their detection in tumor tissue can be used to predict the response of these tumors to treatment with NK1 receptor antagonists (P201330174).

### BRIEF DESCRIPTION OF THE INVENTION

A **first aspect** of the present invention relates to the use of the short (or truncated) form of the NK1 receptor or mRNA that encodes it, with the aim of:
a) diagnosing an individual cancer,
b) monitoring the progress of cancer in an individual,
c) classifying an individual into a stage of disease, and / or
d) predicting response of said individual to cancer treatment.

In a preferred embodiment of this aspect of the invention, the short form (or truncated) NK1 receptor and / or the mRNA which encodes it, is detected in blood, and most preferably in blood plasma. In another preferred embodiment of this aspect of the invention, the diagnosis is an early diagnosis.

In this respect, an early diagnosis of cancer disease or cancer is understood as being when no other symptoms or signs of the disease have been revealed, or those symptoms are not specific or sufficient for diagnosis.

Preferably, the diagnosis can be complementary to other tests, such as, but not limited to, clinical, exploratory, radiological, biochemical (blood or other body fluids) and / or pathological data.

In another preferred embodiment of this aspect of the invention, the cancer treatment response of the individual is predicted, using treatment with NK1 receptor antagonists.

Preferably, the diagnosis can be complementary to other tests, such as, but not limited to, clinical, exploratory, radiological, biochemical (blood or other body fluids) and / or pathological data.

A **second aspect** of the invention relates to an *in vitro* method of obtaining useful data for diagnosing cancer in an individual, hereinafter refrred to as the first method of the invention, comprising detecting expression levels of the short form (or truncated) NK1 receptor, or mRNA which encodes it, in an isolated biological sample from said individual.

In another preferred embodiment, the isolated biological sample is blood, and most preferably, the biological sample is blood plasma from that individual.

In another preferred embodiment, the first method of the invention comprises measuring the ratio between the levels of the long form and truncated form of the NK1 receptor and / or mRNA that encodes it/them.

A **third aspect** of the invention relates to an *in vitro* method for diagnosing an individual with cancer, hereinafter referred to as the second method of the invention, comprising:
a) obtaining a isolated biological sample from the individual,
b) detecting the expression levels of the short (or truncated) NK1 receptor, or mRNA that encodes it, in the biological sample of step (a), and
c) assigning that individual to the group of individuals suffering from cancer when the short form (or truncated) NK1 receptor or its mRNA exhibits superior expression, or overexpression, relative to a reference sample.

In a preferred embodiment of this aspect the first method of the invention further detects expression levels of NK1 receptor or mRNA, which encodes it, in a biological sample from said individual.

In another preferred embodiment, the isolated biological sample is blood, and most preferably, the biological sample is blood plasma from that individual.

In another preferred embodiment, the first method of the invention comprises measuring the ratio between the levels of the long form and the truncated form of the NK1 receptor and / or mRNA that encodes it/them.

In another preferred embodiment, the expression 'superior' or 'overexpression' is defined as:
- A value of the truncated form of the receptor NK1 superior to 0 and / or
- A value of the ratio between the sum of the levels of the long form and the truncated form of the NK1 receptor and another factor which is the result of the level of the long form of said receptor, greater than 1 and / or
- A value of the mRNA which encodes the truncated form of the NK1 receptor greater tan 0.
- A value of the ratio between the level of the mRNA which encodes the truncated form and the level of the mRNA which encodes the long form of said receptor greater than 0.

In another further preferred embodiment, overexpression is defined as an expression level of the truncated form of the NK1 receptor, or mRNA which encodes the truncated form of the NK1 receptor compared to the expression level of the long form of the NK1 receptor, or the mRNA which encodes the long form of the NK1 receptor, respectively, increased by more than at least:
a) 0.01%
b) 0.1%
c) 1%
d) 10%
e) 20%
f) 30%
g) 40%
h) 50%
i) 60%
j) 70%, or
k) 80%
in the isolated biological sample from the individual.

In another preferred embodiment of the present invention, the *in vitro* method of diagnosing cancer in an individual comprises:
a) obtaining an isolated biological sample from the individual,
b) simultaneously detecting expression levels of NK1 receptor and its short or truncated form, or the mRNA that encodes it, in the biological sample of step (a), and
c) assign that individual to the group of individuals suffering from cancer when the ratio between the expression levels of NK1 receptor and expression levels of its truncated form, or the ratio between the mRNA which encodes them respectively, has a value greater than 1.

In another preferred embodiment of this aspect of the invention, the detection of
a. expression levels of the long form of the receptor NK1
b. expression levels of the truncated form of the NK1 receptor
c. the presence of the mRNA which encodes the long form of the NK1 receptor
d. the presence of the mRNA which encodes the truncated form of the NK1 receptor.

It is performed by
i. a process of genetic profiling, such as a microarray, and / or
ii. a method comprising PCR, such as real-time PCR; and/or
iii. Northern blotting, and / or
iv. Western blotting, and / or
iv. an immunohistochemical procedure and / or
v. an ELISA method (Enzyme-Linked ImmunoSorbent Assay)

More preferably, detecting the expression levels of the genes is performed by Q-RT-PCR. In another preferred embodiment, the detection of protein levels of the long form of the NK1 receptor and / or the truncated form of the receptor NK1 is performed by immunological techniques. More preferably, the immunological techniques are based on precipitation reactions, agglutination based reactions, immunostaining, and radioimmunoassay techniques, ELISA *(Enzyme Linked immunoadsorbent Assay),* or any combination thereof. More preferably, the immunological techniques include immunostaining. Even more preferably, the immunostaining is selected from immunostaining with antibodies conjugated with an enzyme, immunostaining with antibodies conjugated with fluorochrome, or cytometry.

More preferably, the biological sample is plasma blood drawn from an individual.

In another preferred embodiment, the cancer is selected from the following: gastric cancer, preferably gastric adenocarcinoma,; colon cancer, preferably colon adenocarcinoma; pancreatic cancer, preferably pancreatic adenocarcinoma; renal cancer, breast cancer, ovarian cancer, preferably ovarian adenocarcinoma; endometrial cancer; uterine cervical cancer; lung cancer, preferably non small-cell lung carcinoma and / or small-cell lung carcinoma; thyroid cancer, papillary thyroid carcinoma preferably and / or follicular thyroid carcinoma; bladder carcinoma, preferably transitional carinoma of urinary bladder; prostate carcinoma; glial lineages cancer of the Central Nervous System (glioma); sarcomas, preferably fibrosarcoma, malignant fibrous histiocytoma and Ewing sarcoma; melanoma; embryonal cancers, preferably neuroblastoma; and hematologic cancers, preferably B - cell or T - cell leukemia, non - Hodgkin lymphomas, preferably B - cell or T - cell, Burkitt's lymphoma, Hodgkin's lymphoma and multiple myeloma.

A **fourth aspect** of the invention relates to an *in vitro* method for classifying an individual suffering from a cancer in a particular tumor stage, hereinafter referred to as the third method of the invention, comprising the steps a) - c) as the first or second method of the invention, and further comprising classifying the individual in the group of individuals suffering from cancer in stage I, II, III or IV depending on:
a. the level of the truncated form of the NK1 receptor and / or
b. level of the mRNA which encodes the truncated form of the NK1 receptor and / or
c. the value of the ratio between the sum of the levels of the long and the truncated form of the NK1 receptor and another factor which is the result of the level of the long form of said receptor and/or
d. the value of the ratio between the level of mRNA which encodes the truncated form and the level of the mRNA which encodes the long form of said receptor.

The classification is based on the criteria in Sobin LH, Gospodarowicz MK, Wittekind Ch. Eds. TNM Classification of Malignant Tumors, 7th ed. Wiley-Blackwell, Oxford 2009; url: http://www.uicc.org/resources/tnm

In a preferred embodiment of this aspect, the third method of the invention further comprises classifying the individual in the group of individuals suffering from cancer in stage I when:
a. the value of the ratio between the sum of the levels of the long and truncated forms of the NK1 receptor and the level of the long form of said receptor, is greater than 1 and less than 1.5 and / or
b. the value of the ratio between the level of mRNA which encodes the truncated form and the level of mRNA which encodes the long form of the receptor is greater than 0 and less than 1.2.

In another preferred embodiment of this aspect, the third method of the invention further comprises classifying the individual in the group of individuals suffering from cancer in stage II when:
a. the value of the ratio between the sum of the levels of the long and truncated forms of the NK1 receptor and the level of the long form of said receptor, is greater than 1, 5 and less than 1.6 and / or
b. the value of the ratio between the level of mRNA which encodes the truncated form and the level of mRNA which encodes the long form of the receptor is greater than 1.2 and less than 1.9.

In another preferred embodiment of this aspect, the third method of the invention further comprises classifying the individual in the group of individuals suffering from cancer in stage III when;
a. the value of the ratio between the sum of the levels of the long and truncated forms of the NK1 receptor and the level of the long form of said receptor, is greater than 1, 6 and less than 1.9 and / or
b. the value of the ratio between the level of mRNA which encodes the truncated form and the level of mRNA which encodes the long form of the receptor is greater than 1.9 and less than 3.9.

In another preferred embodiment of this aspect, the third method of the invention further comprises classifying the individual in the group of individuals suffering from cancer in stage IV when:
a. the value of the ratio between the sum of the levels of the long and truncated forms of the NK1 receptor and the level of the long form of said receptor, is greater than 1.9 and / or
b. the value of the ratio between the level of mRNA which encodes the truncated form and the level of mRNA which encodes the long form of the receptor is greater than 3.9.

In another preferred embodiment of this aspect of the invention, detection of
a. expression levels of the long form of the receptor NK1
b. expression levels of the truncated form of the NK1 receptor
c. the presence of the mRNA which encodes the long form of the NK1 receptor.
d. the presence of the mRNA which encodes the truncated form of the NK1 receptor.

It is performed by
i. a process of genetic profiling, such as a microarray, and / or
ii. a method comprising PCR, such as real-time PCR; and/or
iii. Northern blotting, and / or
iv. Western blotting, and / or
iv. an immunohistochemical procedure and / or
v. an ELISA method.

More preferably the detection of the expression levels of the genes is performed by Q-RT-PCR. In another preferred embodiment, the detection of protein levels of the long form of the NK1 receptor and / or the truncated form of the receptor NK1 is performed using immunological techniques. More preferably, the immunological techniques are based on precipitation reactions, agglutination based reactions, immunostaining, and radioimmunoassay techniques, ELISA *(Enzyme Linked immunoadsorbent Assay),* or any combination thereof. More preferably, the immunological techniques include immunostaining. Even more preferably, the immunostaining is selected from immunostaining with antibodies conjugated with an enzyme, immunostaining with antibodies conjugated with fluorochrome, or cytometry.

More preferably, the biological sample is plasma blood drawn from an individual.

In another preferred embodiment, the cancer is selected from the following: gastric cancer, preferably gastric adenocarcinoma,; colon cancer, preferably colon adenocarcinoma; pancreatic cancer, preferably pancreatic adenocarcinoma; renal cancer, breast cancer, ovarian cancer, preferably ovarian adenocarcinoma; endometrial cancer; uterine cervical cancer; lung cancer, preferably non small-cell lung carcinoma and / or small-cell lung carcinoma; thyroid cancer, papillary thyroid carcinoma preferably and / or follicular thyroid carcinoma; bladder carcinoma, preferably transitional carinoma of urinary bladder; prostate carcinoma; glial lineages cancer of the Central Nervous System (glioma; sarcomas, preferably fibrosarcoma, malignant fibrous histiocytoma and Ewing sarcoma; melanoma; embryonal cancers, preferably neuroblastoma; and hematologic cancers, preferably B - cell or T - cell leukemia, non - Hodgkin lymphomas, preferably B - cell or T - cell, Burkitt's lymphoma, Hodgkin's lymphoma and multiple myeloma.

A **fifth aspect** of the present invention relates to the use of an indicator that is selected from:
a. the level or concentration of the NK1 receptor in blood plasma and / or
b. the level or concentration of the short form (or truncated) NK1 receptor in blood plasma and / or
c. the level or concentration of mRNA which encodes the NK1 receptor in blood plasma and / or
d. the level or concentration of mRNA which encodes the short (or truncated) form of the NK1 receptor in blood plasma and / or
or any combination thereof, to predict or forecast the response of a human subject or individual suffering from cancer, to treatment with an NK1 receptor antagonist. Preferably, the indicators are used simultaneously.

In another preferred embodiment of this aspect of the invention, detection of
a. the expression levels of the long form of the NK1 receptor
b. the expression levels of the truncated form of the NK1 receptor
c. the presence of the mRNA which encodes the long form of the NK1 receptor.
d. the presence of the mRNA which encodes the truncated form of the NK1 receptor.

It is performed by
i. a process of genetic profiling, such as a microarray, and / or
ii. a method comprising PCR, such as real-time PCR;
   I
iii. Northern blotting, and / or
iv. Western blotting, and / or
iv. an immunohistochemical procedure and / or
v. an ELISA method.

More preferably detecting the expression levels of the genes is performed by Q-RT-PCR. In another preferred embodiment, the detection of protein levels of the long form of the NK1 receptor and / or the truncated form of the receptor NK1 is performed by immunological techniques. More preferably, the immunological techniques are based on precipitation reactions, agglutination based reactions, immunostaining, and radioimmunoassay techniques, ELISA *(Enzyme Linked immunoadsorbent Assay),* or any combination thereof. More preferably, the immunological techniques include immunostaining. Even more preferably, the immunostaining is selected from the immunostaining with antibodies conjugated with an enzyme, immunostaining with antibodies conjugated with fluorochrome, or cytometry.

In another preferred embodiment, the cancer is selected from the following: gastric cancer, preferably gastric adenocarcinoma,; colon cancer, preferably colon adenocarcinoma; pancreatic cancer, preferably pancreatic adenocarcinoma; renal cancer, breast cancer, ovarian cancer, preferably ovarian adenocarcinoma; endometrial cancer; uterine cervical cancer; lung cancer, preferably non small-cell lung carcinoma and / or small-cell lung carcinoma; thyroid cancer, papillary thyroid carcinoma preferably and / or follicular thyroid carcinoma; bladder carcinoma, preferably transitional carinoma of urinary bladder; prostate carcinoma; glial lineages cancer of the Central Nervous System (glioma); sarcomas, preferably fibrosarcoma, malignant fibrous histiocytoma and Ewing sarcoma; melanoma; embryonal cancers, preferably neuroblastoma; and hematologic cancers, preferably leukemia B - cell or T - cell, non - Hodgkin lymphomas, preferably B - cell or T - cell, Burkitt's lymphoma, Hodgkin's lymphoma and multiple myeloma.

A **sixth aspect** of the invention relates to an *in vitro* method for evaluating the evolution of an individual diagnosed with cancer comprising detecting NK1 receptor levels and their short (or truncated) form and / or a combination thereof or mRNA which encodes them in a biological sample from said individual, at two or more different times.

In another preferred embodiment of this aspect of the invention, detection of
a. expression levels of the long form of the receptor NK1
b. expression levels of the truncated form of the NK1 receptor
c. the presence of the mRNA which encodes the long form of the NK1 receptor
d. the presence of the mRNA which encodes the truncated form of the NK1 receptor.

It is performed by
i. a process of genetic profiling, such as a microarray, and / or
ii. a method comprising PCR, such as real-time PCR; and/or
iii. Northern blotting, and / or
iv. Western blotting, and / or
iv. an immunohistochemical procedure and / or
v. an ELISA method.

More preferably the detection of the expression levels of the genes is performed by Q-RT-PCR. In another preferred embodiment, the detection of protein levels of the long form of the NK1 receptor and / or the truncated form of the receptor NK1 is performed by immunological techniques. More preferably, the immunological techniques are based on precipitation reactions, agglutination based reactions, immunostaining, and radioimmunoassay techniques, ELISA *(Enzyme Linked immunoadsorbent Assay),* or any combination thereof. More preferably, the immunological techniques include immunostaining. Even more preferably, the immunostaining is selected from the immunostaining with antibodies conjugated with an enzyme, immunostaining with antibodies conjugated with fluorochrome, or cytometry.

More preferably, the biological sample is plasma blood drawn from an individual.

In another preferred embodiment, the cancer is selected from the following: gastric cancer, preferably gastric adenocarcinoma,; colon cancer, preferably colon adenocarcinoma; pancreatic cancer, preferably pancreatic adenocarcinoma; renal cancer, breast cancer, ovarian cancer, preferably ovarian adenocarcinoma; endometrial cancer; uterine cervical cancer; lung cancer, preferably non small-cell lung carcinoma and / or small-cell lung carcinoma; thyroid cancer, papillary thyroid carcinoma preferably and / or follicular thyroid carcinoma; bladder carcinoma, preferably transitional carinoma of urinary bladder; prostate carcinoma; glial lineages cancer of the Central Nervous System (glioma); sarcomas, preferably fibrosarcoma, malignant fibrous histiocytoma and Ewing sarcoma; melanoma; embryonal cancers, preferably neuroblastoma; and hematologic cancers, preferably B - cell or T - cell leukemia, non - Hodgkin lymphomas, preferably B - cell or T - cell, Burkitt's lymphoma, Hodgkin's lymphoma and multiple myeloma.

A **seventh aspect** of the invention relates to a pharmaceutical composition comprising a NK1 agonist in the manufacture of a medicine for the treatment of an individual identifiable by the first, second or third method of the invention, such as individuals suffering from cancer, or who can be classified as being in a particular stage of cancer.

In a preferred embodiment of this aspect of the invention, the antagonist is a non - peptide antagonist. More preferably, the non - peptide antagonist is selected from the list consisting of: aprepitant, Vestipitant, Casopitant, Vofopitant, Ezlopitant, lanepitant, LY-686017, L-733,060, L-732, 138, L-703.606, WIN 62.577, CP -122 721, TAK-637, and R673, CP-100263, WIN 51708, CP-96345, L-760735, CP-122721, L-758298, L-741671, L-742694, CP-99994, T-2328, or any combination thereof. Even more preferably, the non - peptide NK1 receptor antagonist is selected from: Aprepitant, Vestipitant, Casopitant, Vofopitant, Ezlopitant and lanepitant, or any combination thereof.

In another preferred embodiment of this aspect of the invention, the antagonist is a peptide antagonist. More preferably, the peptide antagonist is an antibody or a fragment thereof, specific against NK1, NK2 and / or NK3 cell receptors, or combinations thereof. Even more preferably an antibody or fragment thereof recognizes at least one sequence of NK1, NK2 or NK3 receptors, selected from: SEQ ID NO: 5, SEQ ID NO: 6 or SEQ ID NO: 7, or a fragment thereof, or against amino acid sequences which have a degree of identity with said amino acid sequences of at least 85%, typically at least 90%, preferably at least 95%, more preferably at least 98%, even more preferably at least 99%.

An **eighth aspect** of the invention relates to a kit or device, hereinafter referred to as kit or device of the invention, comprising primers, probes and / or antibodies, or fragments thereof, capable of detecting or binding to the truncated form of the NK1 receptor, or the mRNA which encodes, and preferably also comprising primers, probes and / or antibodies that detect the long form of the NK1 receptor, or the mRNA which encodes it, and where:
- Primers are sequences of polynucleotides of between 10 and 30 base pairs, more preferably between 15 and 25 base pairs, even more preferably between 18 and 22 base pairs, and even more preferably from about 20 base pairs, which have an identity of at least 80%, more preferably at least 90%, even more preferably at least 95%, even more preferably at least 98% and particularly 100% with a fragment of the complementary sequence of SEQ ID NO: 4 (short form of NK1 receptor) and / or SEQ ID NO: 2 (long form of NK1 receptor).
- The probes are polynucleotide sequences of between 80 and 1100 base pairs, more preferably between 100 and 1000 base pairs, and most preferably between 200 and 500 base pairs, which have an identity of at least 80% , more preferably at least 90%, even more preferably at least 95%, even more preferably at least 98%, particularly 100%, with a fragment of the complementary sequences to SEQ ID NO : 4 (short form of the NK1 receptor) and / or SEQ ID NO: 2 (long form of the NK1 receptor),
- The antibodies or fragments thereof are capable of binding to a specific region formed by any of the amino acid sequences SEQ ID NO: 1 (long form of the NK1 receptor), SEQ ID NO: 3 (short form of the NK1 receptor), or a fragment thereof, or against amino acid sequences which have a degree of identity with said amino acid sequences of at least 85%, typically at least 90%, preferably at least 95%, more preferably at least 98%, even more preferably at least 99%.

Preferably oligonucleotides have modifications in some of their nucleotides, such as, but not limited to, nucleotides having any of its atoms with a radioactive isotope, typically ³²P or tritium, nucleotides labeled immunologically, for example with a molecule of digoxigenin, and / or immobilized in a membrane. Several possibilities are known in the state of the art.

In another preferred embodiment, the kit or device of the invention comprises at least one antibody which is selected from:
a) an anti-NK1 antibody in its long form, which will preferably recognize an epitope located at the extreme C-terminal receptor.
b) an anti-NK1 antibody in its truncated form, which will preferably recognize an epitope in one of the transmembrane bridges of the receptor.

More preferably it comprises at least one NK1 anti-antibody in its long form and an NK1 anti- antibody in its truncated form, and most preferably the antibody is monoclonal. In another preferred embodiment, the antibody is polyclonal. Even more preferably, the antibody is labeled with a fluorochrome, and even more preferably the fluorochrome is selected from the list comprising: Fluorescein (FITC), tetramethylrhodamine and derivatives, phycoerythrin (PE), PerCP, Cy5, Texas, allophycocyanin or any combination thereof.

A **ninth aspect** of the invention relates to the use of the kit or device of the invention to carry out a method as described in any of the objectives of the invention.

A **tenth aspect** of the invention relates to a solid support, or protein chip comprising at least one anti-NK1 receptor antibody and / or short form anti - NK1 receptor, or any combination thereof, to carry out any of the methods of the invention.

An **eleventh aspect** of the invention relates to a solid support, or DNA chip, comprising single channel oligonucleotides or microarrays designed from a known sequence or from an mRNA of at least one of the genes (NK1 receptor or truncated receptor).

A **twelfth aspect** of the invention relates to a computer program comprising a program which understands instructions which allow the computer to carry out the method according to any of the methods of the invention.

A **thirteenth aspect** of the invention relates to a storage system readible by a computer program comprising instructions capable of allowing a computer to perform the steps of any of the methods of the invention.

A **fourteenth aspect** of the invention relates to a transmissible signal comprising program instructions capable of allowing a computer to perform the steps of any of the methods of the invention.

### DETAILED DESCRIPTION OF THE INVENTION

As used herein, the terms "level" or "levels" are used when referring to the amount of a substance present and detectable in a given environment. More specifically we use these terms to refer to the amount of a protein like the long or short forms of the NK1 receptor or a nucleic acid sequence such as the mRNA which encodes the short and long forms of said receptor.

The inventor describes herein:
1. The long and short (or truncated) forms of NK1 receptor are present in the blood plasma of patients with cancer.
2. The presence of the short form of the NK1 receptor in the blood plasma is specific to patients with cancer.
3. That the detection of the presence of said short (or truncated) form of the NK1 receptor in blood plasma is possible in patients with cancer.
4. That the detection of the presence of said short (or truncated) form of the NK1 receptor is useful for early diagnosis in early stages of cancer and can be used as a 'screening' method.
5. That the detection of the presence of said short (or truncated) form of the NK1 receptor in the blood plasma of patients with cancer, is useful for monitoring the evolution of the cancer because it increases when the cancer progresses and decreases when these cancerous tumors retract and can be used as way of assessing the evolution of cancerous tumors: growth or progression thereof, response to treatment of these cancers or as an indicator of relapse of the cancerous disease.
6. The detection of the presence of said short form NK1 receptor in the blood plasma of patients with cancer, is useful for predicting response to treatment thereof with antagonists of NK1 receptors.
7. The messenger RNA (mRNA) which encodes the long and short forms (or truncated) of the NK1 receptor is present in the blood plasma of patients with cancer.
8. The presence of Messenger RNA that encodes the short (or truncated) form of the NK1 receptor in the blood plasma is specific to patients with cancer.
9. Detection of messenger RNA, which encodes the short (or truncated) form of the NK1 receptor, is possible in the blood plasma of patients with cancer.
10. That detection of the presence of messenger RNA which encodes the short (or truncated) form of the NK1 receptor in the blood plasma is useful for early diagnosis in early stages of cancer and can be used as a 'screening' method.
11. That detection of the presence of messenger RNA which encodes the short form of the NK1 receptor in the blood plasma of patients with cancer is useful for monitoring the evolution of the cancer because it increases when the cancer progresses and decreases when these cancerous tumors retarct and can be used as way of assessing the evolution of cancerous tumors: growth or progression thereof, response to treatment of these cancers or as an indicator of relapse of the cancerous disease.
12. The detection of the presence of said messenger RNA which encodes the short form of the NK1 receptor in the blood plasma of patients with cancer, is useful for predicting response to treatment with NK1 receptor antagonists.

This knowledge allows for: 1) an earlier diagnosis of tumors even before they become clinically detectable-, 2) the implementation of "screening" in the general population for early detection of cancerous tumors, 3) monitoring the evolution of cancerous tumors, including their response to specific treatments or relapse of the cancer disease, 4) a tool of prognostic importance and 5) the most appropriate choice of treatment guidelines or own support for cancer patients, more effective eventual and less undesirable effects, for a more effective and efficient result.

The present invention relates to the use of an indicator that is selected from:
a. The level of the long form of the NK1 receptor, in one isolated biological sample which, preferably, comprises blood plasma and / or
b. the level of the short form (or truncated) of the NK1 receptor in one isolated biological sample which , preferably, comprises blood plasma and / or
c. level of the mRNA which encodes the long form of NK1 receptor in one isolated biological sample which preferably comprises blood plasma and / or
d. level of the mRNA which encodes the short (or truncated) form of the NK1 receptor and / or, in one isolated biological sample which preferably comprises blood plasma
or any combination thereof, in a human subject, in order to
a. diagnose the presence of cancer,
b. monitor or know the evolution or a cancer so as to determine the progression thereof,
c. monitor or know the response to treatment of a cancer.
d. predict or forecast the response of a cancer treatment with NK1 receptor antagonists.

Preferably, the indicators are used simultaneously. More preferably, the biological sample is peripheral blood plasma.

In the present invention it is shown that in the plasma of the blood plasma of patients suffering from cancer the presence of the NK1 receptor is detectable in their long and short forms (or truncated), as well as the presence of messenger RNA which encodes both forms.

### CANCER DIAGNOSIS METHOD FOR AN INDIVIDUAL

Thus, one aspect of the invention relates to a method for diagnosing cancer in a human subject or individual, which comprises using as an indicator, in an isolated biological sample, preferably comprising the blood plasma of the subject, the presence of the long form (or complete) of the NK1 receptor, or mRNA which encodes it, and / or the level of the short (or truncated) form of the NK1 receptor, or mRNA which encodes it, or a combination thereof.

In a preferred embodiment of this aspect of the invention the level of NK1 receptor in its long form and / or the level of NK1 receptor in short form and / or the level of mRNA which encodes the NK1 receptor and / or encodes the short (or truncated) form of the NK1 receptor and / or the ratio between any of them is used as an indicator.

In another preferred embodiment, the method of the invention comprises the following steps:
a. obtaining an isolated biological sample, preferably it comprises peripheral blood plasma of a human subject.
b. detecting NK1 receptor levels in the long form or mRNA which encodes it, in the sample obtained in step (a).
c. detecting NK1 receptor levels in its short (or truncated) form or the mRNA which encodes it in the sample obtained in step (a).
d. Finding the ratio between the levels detected in any of the steps (b) and (c).

Preferably, the result of the first method of the invention is indicative of the presence of cancer in the human subject from which the sample was obtained, preferably plasma of peripheral blood, although this cancer is not detectable by the current diagnostic methods (clinical, radiological, biochemical, pathological or other).

More preferably,
- the value of the truncated form of NK1 receptor greater than 0 and / or
- the value of the ratio between a factor resulting from the sum of the levels of the long form and the truncated form of the NK1 receptor and another factor which is the level of the complete form of said receptor is greater than 1 and / or
- the value of the mRNA which encodes the truncated form of NK1 receptor is greater than 0 and / or
- the value of the ratio between the level of mRNA which encodes the truncated form and the level of mRNA which encodes the long form of the receptor is greater than 1.

More preferably, the levels of NK1 receptor (in its complete form) or mRNA which encodes it are increased by over 0.01% compared to healthy human subjects in an isolated biological sample obtained in step (a). Even more preferably, the level of NK1 receptor, or mRNA which encodes the NK1 receptor, is increased by over 0.1%, 1%, 10%, 20%, 30%, 40%, 50%, 60%, 70%, 80%, and even more preferably more than 90%. More preferably, the levels of the short (or truncated) form of the NK1 receptor or mRNA that encodes it are increased by more than 0.01% compared to healthy human subjects in an isolated biological sample obtained in step (a) . Even more preferably, the level of the NK1 receptor, or mRNA which encodes the NK1 receptor, is increased by more than 0.1 %, 1%, 10%, 20%, 30%, 40%, 50%, 60%, 70%, 80%, and even more preferably more than 90%.

### METHOD TO ESTABLISH THE LEVEL OF PROGRESSION OF A CANCER

Another aspect of the invention relates to a method for establishing the degree of progression of a cancer in a human subject, wherein the subject suffers from a cancer, comprising using, as an indicator, in an isolated biological sample, preferably, comprising blood plasma from the subject, the presence of the long form of the NK1 receptor and / or the mRNA which encodes it, and / or the level of the short (or truncated) form of the NK1 receptor and / or mRNA which encodes it and/or the ratio between them.

In a preferred embodiment of this aspect of the invention the level of NK1 receptor in its long form and / or the level of NK1 receptor in short form and / or mRNA which encodes the NK1 receptor and / or mRNA which encodes the short (or truncated) form of the receptor and / or the ratio between them is used as an indicator to know or monitor the stage of development of a cancerous tumor in a human subject. Said monitoring comprises determining the level of cancer progression, their evolutionary stage and changes over a period of time. This monitoring can be used to measure the stage of tumor progression, make a prognosis and consequently choose the most appropriate treatment options.

In another preferred embodiment, this method of the invention comprises:
a. obtaining an isolated biological sample comprising periferal plasma blood of a human subject suffering from a cancer.
b. detecting NK1 receptor levels in the long form or mRNA which encodes it, in the sample obtained in step (a).
c. detecting NK1 receptor levels in its short (or truncated) form or the mRNA which encodes it in the sample obtained in step (a).
d. Finding the ratio between the levels detected in steps (b) and (c)
   and
e. repeating steps (a), (b), (c) and (d) periodically over time.

### METHOD FOR MONITORING THE EVOLUTION OF CANCER

Another aspect of the invention relates to a method for monitoring the progression of a cancer in a human subject who is subjected to specific treatment for such cancer, comprising using, as an indicator, in an isolated biological sample, preferably comprising plasma blood drawn from the subject, the presence of the long form of the NK1 receptor and / or the mRNA which encodes it, and / or the level of the short (or truncated) form of the NK1 receptor and / or the mRNA which encodes it and / or the ratio between them.

In a preferred embodiment of this aspect of the invention the level of NK1 receptor in its long form and / or the level of the short form in the NK1 receptor and / or mRNA which encodes the NK1 receptor and / or mRNA which encodes the short (or truncated) form of the receptor and / or the ratio between them is used as an indicator to establish the degree of evolution of a cancerous tumor in a human subject throughout the period of treatment. This monitoring comprises determining the level of cancer progression and its evolutionary stage from the time the specific treatment for the cancer starts. This monitoring can be used to know the degree of response of a tumor to specific treatment and consequently choose the most appropriate treatment options.

In another preferred embodiment, the method of the invention comprises:
a. obtaining an isolated biological sample preferably comprising peripheral blood plasma from the individual,
b. detect NK1 receptor levels in the long form or mRNA which encodes it, in the sample obtained in step (a).
c. detect NK1 receptor levels in its short (or truncated) form or the mRNA which encodes it in the sample obtained in step (a).
d. Find the ratio between the levels of the steps (b) and (c).
   and.
e. Repeat steps (a), (b), (c) and (d) periodically over time during the evolution of the human subject.
f. Find the ratio between the data obtained in each repetition of step (e).

Preferably, the result of this method of the invention is indicative of progression or regression of cancer in the human subject from which the sample was obtained, preferably blood plasma from step (a) and the response to specific treatment. In a preferred embodiment of the third method, the result will be an indicator of cancer progression when the values obtained in the repetitions of step (e) are sequentially higher. In another preferred embodiment of the third method, the result will be an indicator of cancer regression when the values obtained in the repetitions of step (e) are sequentially smaller. In a preferred embodiment of this method, the result will be an indicator of complete regression or complete response to treatment of cancer when the values obtained in repetitions of step (e) are sequentially smaller and the last repetition shows some of the following values:
a. NK1 receptor level is less than 10% of the detectable level of NK1 receptor in healthy subjects (control), and / or
b. level of the short (or truncated) form of the NK1 receptor is less than 1% of the detectable level of the NK1 receptor in healthy subjects (control), and / or
c. level of the short (or truncated) form of the receptor is below 10% of the NK1 receptor level, and / or
d. level of the mRNA which encodes the short (or truncated) form of the NK1 receptor is less than 1% of the mRNA which encodes the short (or truncated) form of the NK1 receptor, and / or
e. any combination thereof.

In a further preferred embodiment of this method, the result will indicate total cancer regression when the values obtained in the repetitions of steps (e) are sequentially smaller and the last repetition shows some of the following values:
a. level of the short (or truncated) form of the NK1 receptor is 0 or undetectable.
b. level of the mRNA which encodes the short (or truncated) form of the NK1 receptor is 0 or undetectable.

In an even more preferred embodiment of the method, the result will be an indicator of relapse or recurrence of the cancer tumor disease when after getting results that indicate total tumor regression or complete response to maintained treatment, the values obtained in repetitions of steps (e) are sequentially larger and the last repetition shows some of the following values:
a. NK1 receptor level is greater than 10% of the NK1 receptor level detectable in healthy subjects (control), and / or
b. level of the short (or truncated) form of the NK1 receptor is greater than 1% of the level of the NK1 receptor detectable in healthy subjects (control), and / or
c. level of the short (or truncated) form of the NK1 receptor is greater than 10% of the level of the NK1 receptor, and / or
d. level of mRNA which encodes the short (or truncated) form of the NK1 receptor greater than 1% of the mRNA which encodes the short (or truncated) form of the NK1 receptor, and / or
e. any combination thereof.

In a further preferred embodiment, the result will be an indicator of relapse or recurrence of the cancer tumor disease when after obtaining results that indicate total tumor regression or complete response to maintained treatment, the values obtained in repetitions of steps (e) are sequentially larger and the last repetition shows some of the following values:
a. level of the short (or truncated) form of NK1 receptor is greater than 0.
b. level of the mRNA which encodes the short (or truncated) form of the NK1 receptor is greater than 0.

In one embodiment, the response is a response to the reduction in tumor burden. In an alternative embodiment, the response is an improvement or absence of deterioration in the state of the tumor. In another embodiment, the response is a clinical outcome such as progression-free survival or overall survival. In another embodiment, the response is an improvement in the size of detectable tumor by radiological techniques, an improvement in the number of detectable tumor cells in blood plasma by immunocytochemistry or flow cytometry, or an improvement in the value of other specific detectable tumor indicators, preferably in blood plasma.

### METHOD TO PREDICT OR FORECAST THE RESPONSE IN CANCER PATIENTS TO TREATMENT WITH A NK1 ANTAGONIST

Another aspect of the invention relates to a method, hereinafter referred to as the fourth method of the invention, to predict or forecast the response to specific treatment with NK1 receptor antagonists, wherein the subject who suffers from cancer, in an isolated biological sample which preferably comprises blood plasma drawn from the subject, which comprises using as an indicator the presence of the long form of the NK1 receptor and / or the mRNA which encodes it, and / or the level of the short (or truncated) form of the NK1 receptor and / or the mRNA which encodes it, and / or the ratio between them.

In a preferred embodiment of this aspect of the invention, the level of the NK1 receptor in its long form and / or the level of the NK1 receptor in short form and / or mRNA which encodes the NK1 receptor and / or mRNA which encodes the short (or truncated) form of said receptor and / or the ratio of these is used as an indicator to predict or forecast the response of a cancer patient to treatment with NK1 antagonists. This embodiment allows, prior to treatment, the prediction of the response of this cancerous tumor to treatment with NK1 receptor antagonists and therefore, faciltates when choosing the most appropriate treatment options. It would also allow for prediction of the response after the treatment has started, and also when choosing if to continue with the same treatment or to choose other therapeutically more suitable options.

In another preferred embodiment, the method of the invention comprises:
a. obtaining an isolated biological sample, preferably comprising peripheral blood plasma of a human subject who suffers from a cancer.
b. detecting NK1 receptor levels in the long form or mRNA which encodes it, in the sample obtained in step (a).
c. detecting NK1 receptor levels in its short (or truncated) form or the mRNA which encodes it in the sample obtained in step (a).
d. Finding the ratio between the levels in steps (b) and (c).

Preferably, the result of the method of the invention is indicative of the response of the cancer in the human subject from which the sample was obtained, preferably blood plasma from step (a), to the specific treatment with NK1 receptor antagonists. In a preferred embodiment of the fourth method, the result will be an indicator of good tumor response to treatment with NK1 receptor antagonists, when in steps (b) and / or (c) and / or (d) some of the following values are displayed:
a. NK1 receptor level is greater than 10% of the detectable level of the NK1 receptor in healthy subjects (control), and / or
b. level of the short (or truncated) form of the NK1 receptor is greater than 1% of the detectable level of the NK1 receptor in healthy subjects (control), and / or
c. level of the short (or truncated) form of the NK1 receptor is greater than 10% of the level of the NK1 receptor, and / or
d. level mRNA which encodes the short (or truncated) form of the NK1 receptor is greater than 1% of the mRNA which encodes the short (or truncated) form of the NK1 receptor, and / or
e. any combination thereof.

In an even more preferred embodiment of the method, the result will indicator total cancer regression values obtained when the repetitions of step (e) are sequentially smaller and the last repetition shows some of the following values:
a. level of the short (or truncated) form of the NK1 receptor is equal to 0 or undetectable.
b. level mRNA which encodes short (or truncated) form of the NK1 receptor is equal to 0 or undetectable .

In one embodiment, the response is a response to the reduction in tumor burden. In an alternative embodiment, the response is an improvement or absence of deterioration in the state of the tumor. In another embodiment, the response is a clinical outcome such as progression-free survival or overall survival. In another embodiment, the response is an improvement in the size of detectable tumor by radiological techniques, an improvement in the number of detectable tumor cells, preferably in blood plasma, by immunocytochemistry or flow cytometry, or an improvement in the value of other specific tumor indicators detectable in blood plasma.

Subjects whose response is predicted are human subjects suffering from cancer. The terms "individual", "human subject", "subject" and "patient" are used interchangeably therefore in this specification.

Herein the term NK1 receptor (also termed neurokinin 1 receptor, SPR; NK1 R; NKIR; or TAC1R) to a G - protein coupled receptor. This is a G protein coupled receptor with seven transmembrane bridges, encoded by the *TAC1R* gene located on chromosome 2 in humans. This gene belongs to a tachykinin receptor gene family. These are characterized tachykinin receptors by interaction with G proteins and contain seven hydrophobic transmembrane regions. This gene encodes for the receptor of the tachykinin substance P, also known as neurokinin 1. The protein encoded is also involved in mediating the metabolism of phosphatidylinositol of substance P.

In the context of the present invention, *TAC1R* is also defined by a nucleotide sequence or polynucleotide, which is the coding sequence of the protein contained in SEQ ID NO: 1 (GeneBank number NP_001049.1), and comprises several variants from:
a) nucleic acid molecules which encode a polypeptide comprising the amino acid sequence of SEQ ID NO: 1,
b) nucleic acid molecules whose complementary strand hybridizes with the polynucleotide sequence of a),
c) nucleic acid molecules whose sequence differs from a) and / or b) due to the degeneration of the genetic code,
d) nucleic acid molecules which encode a polypeptide comprising the amino acid sequence with an identity of at least 80%, 90%, 95%, 98% or 99% to SEQ ID NO: 1, and
wherein the polypeptide encoded by said nucleic acids has the activity and structural characteristics of the protein NK1. Among said nucleic acid molecules, those collected in SEQ ID NO: 2, are found.
SEQ ID NO: 1 
MDNVLPVDSDLSPNISTNTSEPNQFVQPAWQIVLWAAAYTVIVVTSVVGNVVVMWIIL
AHKRMRTVTNYFLVNLAFAEASMAAFNTVVNFTYAVHNEWYYGLFYCKFHNFFPIAA
VFASIYSMTAVAFDRYMAIIHPLQPRLSATATKVVICVIWVLALLLAFPQGYYSTTETMP
SRVVCMIEWPEHPNKIYEKVYHICVTVLIYFLPLLVIGYAYTVVGITLWASEIPGDSSDR
YHEQVSAKRKVVKMMIVVVCTFAICWLPFHIFFLLPYINPDLYLKKFIQQVYLAIMWLAM
SSTMYNPIIYCCLNDRFRLGFKHAFRCCPFISAGDYEGLEMKSTRYLQTQGSVYKVSR
LETTISTVVGAHEEEPEDGPKATPSSLDLTSNCSSRSDSKTMTESFSFSSNVLS
SEQ ID NO: 2

agctgagcaacccgaaccgagaggtgcccgcgaaactgcaggcggcggcagcggcagcaaagagaaggaaa
aatctccagctggatacgaagctccagaatcctggccataggctcagaacttttacaggtcgcgctgcaatggcccccca
cttcgctcctaagtcctcacgcagcacagggctttgcctttccctgcggaggaaggagaaataggagttgcaggcagca
gcaggtgcataaatgcgggggatctcttgcttcctagaactgtgaccggtggaatttctttcccttttttcagtttaccgcaaga
gagatgctgtctccagacttctgaactcaaacgtctcctgaagcttgaaagtggaggaattcagagccaccgcgggcag
gcgggcagtgcatccagaagcgtttatattctgagcgccagttcagctttcaaaaagagtgctgcccagaaaaagccttc
caccctcctgtctggctttagaaggaccctgagccccaggcgccagccacaggactctgctgcagaggggggttgtgta
cagatagtagggctttaccgcctagcttcgaaatggataacgtcctcccggtggactcagacctctccccaaacatctcca
ctaacacctcggaacccaatcagttcgtgcaaccagcctggcaaattgtcctttgggcagctgcctacacggtcattgtggt
gacctctgtggtgggcaacgtggtagtgatgtggatcatcttagcccacaaaagaatgaggacagtgacgaactattttct
ggtgaacctggccttcgcggaggcctccatggctgcattcaatacagtggtgaacttcacctatgctgtccacaacgaatg
gtactacggcctgttctactgcaagttccacaacttctttcccatcgccgctgtcttcgccagtatctactccatgacggctgtg
gcctttgataggtacatggccatcatacatcccctccagccccggctgtcagccacagccaccaaagtggtcatctgtgtc
atctgggtcctggctctcctgctggccttcccccagggctactactcaaccacagagaccatgcccagcagagtcgtgtgc
atgatcgaatggccagagcatccgaacaagatttatgagaaagtgtaccacatctgtgtgactgtgctgatctacttcctcc
ccctgctggtgattggctatgcatacaccgtagtgggaatcacactatgggccagtgagatccccggggactcctctgac
cgctaccacgagcaagtctctgccaagcgcaaggtggtcaaaatgatgattgtcgtggtgtgcaccttcgccatctgctgg
ctgcccttccacatcttcttcctcctgccctacatcaacccagatctctacctgaagaagtttatccagcaggtctacctggcc
atcatgtggctggccatgagctccaccatgtacaaccccatcatctactgctgcctcaatgacaggttccgtctgggcttca
agcatgccttccggtgctgccccttcatcagcgccggcgactatgaggggctggaaatgaaatccacccggtatctccag
acccagggcagtgtgtacaaagtcagccgcctggagaccaccatctccacagtggtgggggcccacgaggaggagc
cagaggacggccccaaggccacaccctcgtccctggacctgacctccaactgctcttcacgaagtgactccaagacca
tgacagagagcttcagcttctcctccaatgtgctctcctaggccacagggcctttggcaggtgcagcccccactgcctttga
cctgcctcccttcatgcatggaaattcccttcatctggaaccatcagaaacaccctcacactgggacttgcaaaaagggtc
agtatgggttagggaaaacattccatccttgagtcaaaaaatctcaattcttccctatctttgccaccctcatgctgtgtgactc
aaaccaaatcactgaactttgctgagcctgtaaaataaaaggtcggaccagcttttcccaaaagcccattcattccattctg
gaagtgactttggctgcatgcgagtgctcatttcaggatgaattctgcagcacagctgcggacccggaagactcattttcct
ggagccccgtgttacttcaataaagttatctcagattagcctcctgcagctggaggctcctatcaccccagcctacgcttga
cagggtgaacaaaagaaggcaccacataacatctaaatgaaaaatttagccctgtcttctaagcatctgtgaaaagaaa
catatgtattccccttttttggcatctcagtatttcagtacatttatacatcatgagattgagaacctcgggcttccacattatgtcc
ccggtgactgtcctgagcagccgacgcaagcagaatatgtccactgatacctgctagttctcttacagaccaggaattgg
gagacttgcactacatttaatgtgtagttgaccctcttttcctacttgtaaacaaggggactgaactagataatctaagtgttcc
ttcgaatcttaacatcccgtggttcaaggattgtatgagtttttttgttgttttacaaaaaaaaacaaaacgaagaataaaaga
atagaaaagaataggagcagtgagtcttgtaactaatacccagttcctggagatgtagcaactgctaaggccatctgtaa
ctatccatctcagacattctccgatttatcttaaaatcctgagtacattccttctcatggaaggttttggcttttgacagagcaga

ggacttcatgccaaggcctgcatccatccagctttagcaggcagaatttcatagctgcagaacactgtcagagaagaca
aatgtgggctccctgctttaaccttttgggtattttagggtgggggccctaaccttcattcttagttttacactagcatcgtgctcat
atgtgcgacaagcaagaaggctgcactttgcagctgcacttctgggaagagggcatcttgcatcttcccttcagactctctg
aatgtctcctccctgctccatggctttgccagcttcctgtctctaaggggtagaatgactcatcaaccctaaaggacagtcag
tcttccaagagccatgaactgaatgctttatatcctaatttagatttagagtttccagaaggtgagcatgcagttttgttttgtttttt
tttctgtctcccaaatctgtgtttttccagatatggctggaagcagaagcttcatgtaacatccatgaatgtcctcctggtagttt
gcataatggatgcacatgtgccgcatccataacattaaggggagaataatgcatggtttacagcctttgccagccctgctg
gctctaattctaccagggcatccacaggcctgggggaagaagaaacagtataagccagaaaacctcaagaactacat
tctctaaagcagcatggaaagttttaaataaactaagtgaagccagatcattgcagatatataaatggaagacaaaattta
gaagcaacaaaagttagtgccctaagcattagtcatacttccaatagagaatcttgctgtgtatggattactcactttggaag
aatgtaaagagctaacatgattatgagaagtacctgagaagatggtgtcaagaagttggggacaccccatctatggaag
agaaggttagagttgagctcaacgaggattaactgagtgcctcctctggactttgccctgaactgggaacacacagcccc
tgcagctcttgaagagcctaccttattggccatcactaactaactcaccagtcctagtgagtctaagctgcccagcagtcttg
gaggcatctgagaggacagattctccacagaattctaaaaacccacactcaacatgggcagtcaagccaaagactgg
gacctttggagagcctctggaatgagagttctctggggtacttccaaagggagctggcagtcagtccaggggacctaaa
ggaatttggttgaacagtatcatctctgtgcatagtaagagggaatgttgggtggtccgggcagtttccaatatggcaaagc
atctgcttggacagtgccagcaagccttcctctgacccagtctccaatgtccactaacttataaaaatgtcatcaactccca
catgtaagaaacaccatgatttgtactgtgcatgggtcacattcttattctagaaatgcatcaccctgtgtttatccaagtgtgtt
tacttggtgtaatgtccagtagtaatagaatatgaaatatcaaggaaccatctttgttacgtgacttccaaaatgtgagatctc
attgctgtcactgtgatatttgtattgtgtgaatctcttcctcctcttcctcctcatgctttctcagggaggagccctgatgtatatca
tgaactcacagttcctagaccacagtaattgaggggcggtggggggcctttatcggagaagctagagaacaagagtc
cttctcctccttatcccccaacaggacactaagagacaaggactgagtggaatcctggagaaaggggactcaggaact
gacctcattggcctgatttgtgaggagaggagtataagtggagaggggccattcctgaggtttccgtgttttccagcctggtc
tcctggaaagaatctttatacagaaataaagtatgtgtttcactcaaaaaaaaaaaaaaaa

The short (or truncated) form of the receptor is collected, but not limited to, in GeneBank sequences NP_056542.1 (SEQ ID NO: 3) and NM_015727.2 (SEA ID NO: 4), or amino acid and nucleotide sequences, respectively, which have an identity of at least 60%, 80%, 90%, 95%, 98% or 99% with SEQ ID NO: 3 or SEQ ID NO: 4, respectively.
SEQ ID NO: 3 
MDNVLPVDSDLSPNISTNTSEPNQFVQPAWQIVLWAAAYTVIVVTSVVGNVVVMWIIL
AHKRMRTVTNYFLVNLAFAEASMAAFNTVVNFTYAVHNEWYYGLFYCKFHNFFPIAA
VFASIYSMTAVAFDRYMAIIHPLQPRLSATATKVVICVIWVLALLLAFPQGYYSTTETMP
SRVVCMIEWPEHPNKIYEKVYHICVTVLIYFLPLLVIGYAYTVVGITLWASEIPGDSSDR
 
YHEQVSAKRKVVKMMIVVVCTFAICWLPFHIFFLLPYINPDLYLKKFIQQVYLAIMWLAM
SSTMYNPIIYCCLNDR
SEQ ID NO: 4 
agctgagcaacccgaaccgagaggtgcccgcgaaactgcaggcggcggcagcggcagcaaaagagaaggaaa
aatctccagctggatacgaagctccagaatcctggccataggctcagaacttttacaggtcgcgctgcaatgggcccca
cttcgctcctaagtcctcacgcagcacagggctttgcctttccctgcggaggaaggagaaataggagttgcaggcagca
gcaggtgcataaatgcgggggatctcttgcttcctagaactgtgaccggtggaatttctttcccttttcagtttaccgcaaga
gagatgctgtctccagacttctgaactcaaacgtctcctgaagcttgaaagtggaggaattcagagccaccgcgggcag
gcgggcagtgcatccagaagcgtttatattctgagcgccagttcagctttcaaaaagagtgctgcccagaaaaagccttc
cacccctcctgtctggctttagaaggaccctgagccccaggcgccagccacaggactctgctgcagagggggttgtgta
cagatagtagggctttaccgcctagcttcgaaatggataacgtcctcccggtggactcagacctctccccaaacatctcca
ctaacacctcggaacccaatcagttcgtgcaaccagcctggcaaattgtcctttgggcagctgcctacacggtcattgtggt
gacctctgtggtgggcaacgtggtagtgatgtggatcatcttagcccacaaaagaatgaggacagtgacgaactattttct
ggtgaacctggccttcgcggaggcctccatggctgcattcaatacagtggtgaacttcacctatgctgtccacaacgaatg
gtactacggcctgttctactgcaagttccacaacttctttcccatcgccgctgtcttcgccagtatctactccatgacggctgtg
gcctttgataggtacatggccatcatacatcccctccagccccggctgtcagccacagccaccaaagtggtcatctgtgtc
atctgggtcctggctctcctgctggccttcccccagggctactactcaaccacagagaccatgcccagcagagtcgtgtgc
atgatcgaatggccagagcatccgaacaagatttatgagaaagtgtaccacatctgtgtgactgtgctgatctacttcctcc
ccctgctggtgattggctatgcatacaccgtagtgggaatcacactatgggccagtgagatccccggggactcctctgac
cgctaccacgagcaagtctctgccaagcgcaaggtggtcaaaatgatgattgtcgtggtgtgcaccttcgccatctgctgg
ctgcccttccacatcttcttcctcctgccctacatcaacccagatctctacctgaagaagtttatccagcaggtctacctggcc
atcatgtggctggccatgagctccaccatgtacaaccccatcatctactgctgcctcaatgacaggtgaggatcccaacc
ccatgagctctccaggggccacaagaccatctacatacacagtggccaagcggccatcctaaatgagtaaacccagct
gtgagacaagagggacaagtggggactgcagctaacttatcatcacacaactcagcctggctgattatcaccatccagg
aatgggagcccggagtggactgattttctttttttcttttcca
SEQ ID NO: 5: 
MDNVLPVDSDLSPNISTNTSEPNQFVQPAWQIVLWAAAYTVIVVTSVVGNVVVMWIIL
AHKRMRTVTNYFLVNLAFAEASMAAFNTVVNFTYAVHNEWYYGLFYCKFHNFFPIAA
VFASIYSMTAVAFDRYMAIIHPLQPRLSATATKVVICVIWVLALLLAFPQGYYSTTETMP
SRVVCMIEWPEHPNKIYEKVYHICVTVLIYFLPLLVIGYAYTVVGITLWASEIPGDSSDR
YHEQVSAKRKVVKMMIVVVCTFAICWLPFHIFFLLPYINPDLYLKKFIQQVYLAIMWLAM
 
SSTMYNPIIYCCLNDRFRLGFKHAFRCCPFISAGDYEGLEMKSTRYLQTQGSVYKVSR
LETTISTVVGAHEEEPEDGPKATPSSLDLTSNCSSRSDSKTMTESFSFSSNVLS
SEQ ID NO: 6 
MGTCDIVTEANISSGPESNTTGITAFSMPSWQLALWATAYLALVLVAVTGNAIVIWIILA
HRRMRTVTNYFIVNLALADLCMAAFNAAFNFVYASHNIWYFGRAFCYFQNLFPITAMF
VSIYSMTAIAADRYMAIVHPFQPRLSAPSTKAVIAGIWLVALALASPQCFYSTVTMDQG
ATKCVVAWPEDSGGKTLLLYHLVVIALIYFLPLAVMFVAYSVIGLTLWRRAVPGHQAH
GANLRHLQAMKKFVKTMVLVVLTFAICWLPYHLYFILGSFQEDIYCHKFIQQVYLALFW
LAMSSTMYNPIIYCCLNHRFRSGFRLAFRCCPWVTPTKEDKLELTPTTSLSTRVNRCH
TKETLFMAGDTAPSEATSGEAGRPQDGSGLWFGYGLLAPTKTHVEI
SEQ ID NO: 7

In the context of the present invention, "reference sample" or "reference value" is understood to be the sample used to determine the variation in expression levels of proteins or nucleic acids in the context of the the present invention. In one embodiment of the invention, the reference value is obtained from the signal provided using a tissue sample obtained from an individual who does not have a tumor. Preferably, samples are taken from blood plasma from several individuals with and without cancer and combined, so that the reference value reflects the average value of said molecules in the population of individuals with and without cancer. "Reference value" is the level of a protein of the invention (NK1 receptor, or truncated form NK1 receptor) or nucleic acids of the present invention (mRNA of the NK1 gene and mRNA of the truncated form of said receptor) in the reference sample.

In the present invention, "prognosis" is defined as the expected evolution of the disease and refers to assessing the probability according to which a subject suffering from a disease as well as the assessment of its beginning stage, stage of development, evolution, or its regression, and / or prognosis of disease course in the future. As those experts in this field will understand, such an assessment may usually not be correct for 100% of the subjects to be diagnosed, although we would prefer it to be. The term, however, requires that a statistically significant portion of subjects can be identified as suffering from the disease or are genetically predisposed to it. If one part is statistically significant it can be determined quickly by the experts in this field using various well-known statistical evaluation tools, for example, to determine confidence intervals, determine p values , Student's t test, Mann-Whitney test , etc. Preferred confidence intervals are at least 50%, at least 60%, at least 70%, at least 80%, at least 90%, at least 95%. P-values are, preferably, 0.2, 0.1, 0.05.

"Predicting the response" is understood, in the context of the present invention, as determining the probability that the patient responds favorably or unfavorably to a particular therapy or treatment, including surgical treatment. Especially, the term "prediction" as used herein refers to an individual assessment of any parameter that can be useful in determining the evolution of a patient. As understood by the experts in this field, predicting the clinical response to treatment, although it is preferred, may not be correct for 100% of the subjects to be diagnosed or assessed. The term, however, requires you to be able to identify a statistically significant proportion of subjects who have an increased probability of having a positive response. An expert in this field can readily determine whether a subject is statistically significant using various well-known statistical evaluation tools, for example, determining confidence intervals, determining the values of P, Student's t test, Mann-Whitney test, etc . Preferred confidence intervals are at least 50%>, at least 60%>, at least 70%>, at least 80%>, at least 90%), at least 95%>. P-values are, preferably, 0.2, 0.1 or 0.05. Predicting the clinical response can be done using any evaluation criteria used in oncology and known to the expert in this field. In turn, considering the method of the present invention, other subcategories could be established within the main category, thereby facilitating the choice and setting appropriate therapeutic or treatment regimes. This discrimination, as is understood by the experts, is not intended to be correct for 100% of the samples tested. However, it requires that a statistically significant number of samples analyzed are classified correctly. The amount that is statistically significant can be established by an expert in the field using different statistical tools, for example, but not limited to determining confidence intervals, determining the significance value of P, Student's t test or Fisher's discriminant functions, Mann Whitney's nonparametric measures, Spearman's correlation, logistical regression, linear regression, the area under the ROC curve (AUC). Preferably, the confidence intervals are at least 90%, at least 95%, at least 97%, at least 98% or at least 99%. Preferably, the value of p is less than 0.1, 0.05, 0.01, 0.005 or 0.0001. Preferably, the present invention enables the correct detection of the disease differentially in at least 60%, more preferably at least 70%, more preferably at least 80%, or even more preferably at least 90% of the subjects of a certain group or population analyzed.

In the context of the present invention, the term "positive response" refers to a more effective response by cancer patients to treatment with NK1 receptor antagonists, than other cancer patients who did not respond effectively to treatment with NK1 receptor antagonists. The response may refer to the characteristics of tumors of the subject. Alternatively but not mutually exclusively, the answer may refer to the clinical outcome of the subject. Thus, using the first method of the invention, one can predict whether an individual patient displays (i) a response (R) to treatment with NK1 receptor antagonists or (ii) no response (NR) to treatment with antagonists NK1 receptor.

Whenever the answer refers to the characteristics of the tumor of the subject, the distinction between R and NR can be carried out on the basis of change in lesion size. For example, you can define "Response" as a decrease in total tumor burden (defined as the sum of the areas of tumor lesions measured bidimensionally) with reference values corresponding to the initial value, without the appearance of a new lesion. In particular, the answer may be a ≥ 5% decrease in total tumor burden, as well as as a ≥ 10% decrease in total tumor burden, a ≥ 15% decrease in total tumor burden, a ≥ 20% decrease in total tumor burden, a ≥ 25% decrease in total tumor burden, a ≥ 30% decrease in total tumor burden, a ≥ 35% decrease in total tumor burden, a ≥ 40% decrease in total tumor burden, a ≥ 45% decrease in total tumor burden or a ≥ 50% decrease in total tumor burden, each defined as the sum of the areas of tumor lesions measured bidimensionally. In a preferred embodiment of the present invention, the response is defined as a ≥ 30% decrease in total tumor burden (defined as the sum of the tumor areas of the lesions measured bidimensionally) with reference values corresponding to the initial values, without the appearance of a new lesion. Non-response (NR) is defined as contrary to (R). Preferably, non-response (NR) was defined as a <30% decrease in the tumor area of one or more measurable lesions or the appearance of at least one new lesion. This typically includes an increase of > 20% increase in the area of one or more measurable lesions or the appearance of at least one new lesion.

Whenever "response" refers to the clinical outcome of the subject, we can express the "Response" as overall survival or progression-free survival. The survival of cancer patients is generally expressed adequately by the Kaplan-Meier curves, which received their name from Edward L. Kaplan and Paul Meier who were the first to describe them (Kaplan Meier:Amer. Statist . Assn. 53: 457- 35 481).

Furthermore, in the present invention it is demonstrated that patients with cancerous tumors that release a greater amount of the truncated form of the NK1 receptor and / or an increased amount of mRNA which encodes said truncated form of the receptor in the fluid medium in which they are contained, which may be the plasma of peripheral blood plasma, have a better (more effective) response to treatment with NK1 receptor antagonists.

Therefore, in another preferred embodiment of this aspect of the invention, in the fourth method, the presence or absence of the truncated form of the NK1 receptor in the biological sample is also determined, preferably blood plasma sample from step (a), of cancer patients, with the result indicative of a positive response (more effective response to treatment with antagonists of NK1 receptors) if in said blood plasma the truncated form of the NK1 receptor is detected, or mRNA which encodes it.

The expression levels of the genes will give a specific gene expression profile. The term 'level', 'expression level' also called "gene product amount" refers to biochemical materials, either RNA or protein resulting from gene expression. Sometimes a measurement of the amount of gene product is used to infer how active a gene is. "Gene expression profile" is understood as the gene profile obtained after quantification of mRNA and / or protein produced by the gene of interest or bioindicators, that is, by the genes used as biological indicators in the present invention, in an isolated biological sample. The gene expression profile is preferably performed by determining the level of mRNA derived from their transcription, after total extraction of RNA present in the isolated biological sample, which can be done using protocols known in the state of the art. Determining the level of mRNA derived from the transcription of genes used as biological indicators in the present invention, may be carried out, for example, although not limited to, by amplification using polymerase chain reaction (PCR), reverse transcription in combination with the polymerase chain reaction (RT-PCR), quantitative RT-PCR, reverse transcription in combination with ligase chain reaction (RT-LCR), or any other method of nucleic acid amplification; serial analysis of gene expression (SAGE SuperSAGE); DNA chips made with oligonucleotides deposited by any mechanism; DNA microarrays made with oligonucleotides synthesized *in situ* by photolithography or any other mechanism; in situ hybridization using specific probes labeled using any labeling method; by electrophoresis gels; by transfer to membrane and hybridization with a specific probe; by nuclear magnetic resonance or other imaging techniques using paramagnetic diagnostic nanoparticles or any other type of detectable nanoparticles functionalized with antibodies or by any other means. The gene expression profile may also be obtained by detecting and / or quantifying the product of translation, for example, proteins, of mRNA derived from the transcription of genes used as biological indicators in the present invention, using, but not limited to, immunodetection by Western blotting. Quantitative detection of the gene expression used as biological indicators in the present invention can be accomplished more preferably by real-time PCR (RT-PCR or RTqPCR). The real time detection of amplified products can be accomplished using fluorescent molecules intercalated into dsDNA (double stranded DNA) or by hybridization with different probes.

Thus, detection of protein levels or gene expression levels can be carried out by any of the techniques known to the expert in this field. Thus, in another preferred embodiment of this aspect of the invention,
a. the detection of expression levels of NK1 receptor or mRNA which encodes the NK1 receptor in the sample of blood plasma obtained in step (a).
b. the detection of expression levels of short (or truncated) form of the receptor NK1 in the sample of blood plasma obtained in step (a).

It is performed by
i. a process of genetic profiling, such as a microarray and / or
ii. a method comprising PCR, such as real-time PCR and / or
iii. Northern blotting and / or
iv. Western blotting and / or
v. the ELISA procedure.

In another preferred embodiment, the biological sample is blood plasma or RNA extracted from a fluid drawn from a cancer patient. More preferably, the first method of the invention is carried out *in vitro* using a parent sample of the human subject.

In another preferred embodiment, the detection of the gene expression levels is performed by Q-RT-PCR.

In another preferred embodiment, the detection of protein levels and NK1 / or the truncated receptor of NK1, is performed by immunological techniques. In a more preferred embodiment, the immunological techniques are based on precipitation reactions, agglutination based reactions, immunostaining, and radioimmunoassay techniques radioinmunometricas, ELISA (*Enzyme Linked Assay immunoadsorbent*), or any combination thereof. In another more preferred embodiment, the immunological techniques include immunostaining. In another further preferred embodiment, the immunostaining is selected from immunostaining antibodies conjugated with enzymes, immunostaining antibodies conjugated with fluorochrome - or cytometry. Even more preferably, the cytometry is flow cytometry.

In another preferred embodiment of this aspect of the invention, the individual or human subject suffers from one of the following cancers: gastric cancer, preferably gastric adenocarcinoma, colon cancer, preferably colon adenocarcinoma, pancreatic cancer, preferably pancreatic adenocarcinoma, kidney cancer, preferably clear cell renal carcinoma, breast cancer, preferably breast adenocarcinoma, ovarian cancer, preferably ovarian adenocarcinoma, endometrial carcinoma, carcinoma of the uterine cervix, lung cancer, preferably non small-cell lung carcinoma and / or small-cell lung carcinoma, thyroid cancer, preferably papillary thyroid carcinoma and / or follicular thyroid carcinoma, bladder carcinoma, preferably transitional cell carcinoma of the urinary bladder, prostate carcinoma, glial lineages cancer of the Central Nervous System (gliomas, astrocytomas, ependymomas), sarcomas, preferably fibrosarcoma, malignant fibrous histiocytoma and Ewing sarcoma, melanoma, embryonal cancers, preferably neuroblastoma (also hepatoblastoma, medulloblastoma, retinoblastoma, nephroblastoma) and hematological cancers, preferably B-cell or T-cell leukemia, non-Hodgkin lymphomas, preferably B-cell or T-cell, Burkitt's lymphoma, Hodgkin's lymphoma and multiple myeloma.

In another preferred embodiment of this aspect of the invention, the NK1 receptor antagonists are non-peptide antagonists, and most preferably, the non-peptide antagonists are selected from: Aprepitant (or MK 869 or L-754 030), Vestipitant (or GW597599), Casopitant (or GW679769), Vofopitant (or GR-205171), Ezlopitant (or CJ -11,974), lanepitant (or LY-303870), LY-686017, L-733,060 ((2S, 3S) -3 - [(3,5-bis (trifluoromethyl) phenyl) methoxy] -2-phenylpiperidine hydrochloride), L-732.138 (N-Acetyl-L-tryptophan 3,5-bis (trifluoromethyl) benzyl ester), L-703.606 (cis-2-(diphenylmethyl) -N - [(2-iodophenyl) methyl] -1-azabicyclo [2.2.2] octan-3-amine oxalate salt), WIN 62.577, CP-122721, TAK-637, R673, CP-100263, WIN 51708, CP-96345, L-760735, CP-122721, L-758298, L-741671, L-742694, CP-99994, T-2328, or any combination thereof. Compounds preferred are: Aprepitant or MK-869 or L 754030 (MSD), or Vestipitant GW597599 (GSK) and Casopitant or GW679769 (GSK), Fosaprepitant (MSD), or any combination thereof.

In another preferred embodiment of this aspect of the invention, NK1 receptor antagonists are peptide antagonists, and still more preferably, are selected from antibodies or fragments of the agonists which are directed against the NK1 receptor.

As used herein "non-peptide antagonist of NK1 receptors" means any substance of a non-peptide nature of sufficient size and composition to be suitable for binding to the NK1 receptor and thereby inhibit its normal operation, including preventing substance P or other agonists of these receptors from binding to said receptors.

Antibodies are peptidic compounds with the capacity to bind highly selectively to various organic molecules. Binding to specific cellular receptors induces a change in the activity of said receptors, which can determine a change in the normal physiological activity of cellular metabolism.

As used herein "antibody against NK1 receptors" means any polyclonal or monoclonal antibody or fragments of said antibodies against NK1 receptors. An antibody fragment means a part of an antibody against NK1 receptors which is of sufficient size and composition to be suitable for binding to an epitope present on NK1 receptors and thus modify normal operation, including preventing substance P or other agonists of these receptors from binding to said receptors.

The use of monoclonal antibodies directed against the extracellular domain of various cell receptors, such as cancer treatment, is widespread in current practise. Among these therapeutic monoclonal antibodies approved for use in oncology, trastuzumab, which is an anti-ErbB2 / HER2 for breast cancer, cetuximab, which is an anti-ErbB1 / EGFR for colon cancer, and bevacizumab, which is a anti-VEGFR receptor (derived growth factor vascular endothelial) for various types of cancers (Adams *et al.,* 2005) are found.

"Cancer" is understood to be a malignant tumor of unlimited growth potential that expands locally by invasion and systemically by metastasis. According to the present invention, the non-peptide NK1 receptor antagonist is administered to individuals with cancer.

In the context of the present invention, "medicines aimed at modifying the operation of the NK1 receptor" refer to non-peptide antibodies or antagonist molecules, or fragments thereof, which act on the NK1 receptor and modify its operation are used in order to produce inhibition of proliferation, death and / or apoptosis in tumor cells, in a mammal, including humans, either by direct action on the physiological mechanisms of tumor cells or by modifying the tumor microenvironment.

In the context of the present invention, the disease is cancer, preferably gastric cancer, preferably gastric adenocarcinoma, colon cancer, preferably colon adenocarcinoma, pancreatic cancer, preferably pancreatic adenocarcinoma, renal cancer, preferably clear-cell renal carcinoma, breast cancer, preferably breast adenocarcinoma and / or breast carcinoma, ovarian cancer, preferably ovarian adenocarcinoma and / or ovarian carcinoma, endometrial carcinoma, carcinoma of the uterine cervix, lung cancer, preferably lung adenocarcinoma, non small-cell lung carcinoma and / or small-cell lung carcinoma, thyroid cancer, preferably metastatic papillary thyroid carcinoma and / or follicular thyroid carcinoma, bladder cancer, preferably carcinoma of urinary bladder and / or transitional-cell carcinoma of the urinary bladder, prostate carcinoma tumor glial lineages cancer of the Central Nervous System (glioma), sarcomas, preferably fibrosarcoma, malignant fibrous histiocytoma and Ewing sarcoma (although it is also useful in human endometrial stromal sarcoma, osteosarcoma and / or rhabdomyosarcoma), melanoma, embryonal cancers, preferably neuroblastoma (although it is also useful in medulloblastoma, retinoblastoma, nephroblastoma and / or hepatoblastoma) and haematological cancers, preferably B-cell or T-cell leukemia, non-Hodgkin lymphomas, preferably B-cell or T-cell, Burkitt's lymphoma, Hodgkin's lymphoma, leukemias, preferably B-cell or T-cell, multiple myeloma.

Chlorambucil, Melphalan, Aldesleukin, 6-mercaptopurine, 5: these NK1 receptor antagonists, both peptide and nonpeptide, may be used combined among themselves or with various anticancer agents that are selected, but not limiting, from any of the following -fluoruracilo, Ara-c, Bexarotene, Bleomycin, Capecitabine, Carboplatin, Cisplatin, Docetaxel, Doxorubicin, Epirubicin, fludarabine, irinotecan, Methotrexate, Mitoxantrone, Oxaliplatin, Paclitaxel, Rituximab etoposide, teniposide, vincristine, vinblastine, vinorelbine, Imatinib, Erlotinib , cetuximab, trastuzumab, erlotinib, dasatanib, nilotinib, decatanib, panitumumab, amrubicin, oregovomab, Lep-ETU, nolatrexed, AZD2171, batabulin, ofatumumab, zanolimumab, edotecarin, tetrandrine, rubitecan, tesmilifene, oblimersen, Ticilimumab, ipilimumab, gossypol, Bio 111, 131-I-TM-601, ALT-110, BIO 140, CC 8490, cilengitide, gimatecan, IL13-PE38QQR, INO 1001, IPDR, KRX-0402, lucanthone, LY 317615, neuradiab, vitespan, Rte 744, Sdx 102, Talampanel, atrasentan, Xr 311, everolimus, trabectedin, Abraxane, TLK 286, AV-299, DN-101, pazopanib, GSK690693, RTA 744, ON0910.Na, AZD 6244 (ARRY-142886), AMN-107, TKI -258, GSK461364, AZD 1152, enzastaurin, vandetanib, ARQ-197, MK-0457, MLN8054, PHA-739358, R-763, AT-9263, or any combination thereof.

### MEDICAL USES OF THE INVENTION

A **seventh aspect** of the invention relates to a pharmaceutical composition comprising an antagonist of the NK1 receptor in the manufacture of a medicine for the treatment of an individual identifiable by the first, second or third method of the invention, such as individuals with cancer, or who can be classified in a particular stage of cancer.

In a preferred embodiment of this aspect of the invention, the NK1 receptor antagonist is a non-peptide antagonist. More preferably, the non-peptidic antagonist is selected from a list consisting of: aprepitant, Vestipitant, Casopitant, Vofopitant, Ezlopitant, lanepitant, LY-686017, L-733,060, L-732, 138, L-703.606, WIN 62.577, CP -122 721, TAK-637, and R673, CP-100263, WIN 51708, CP-96345, L-760735, CP-122721, L-758298, L-741671, L-742694, CP-99994, T-2328, or any combination thereof. Even more preferably, the non-peptide antagonist of the NK1 recepetor is selected from: Aprepitant, Vestipitant, Casopitant, Vofopitant, Ezlopitant and lanepitant, or any combination thereof.

In another preferred embodiment of this aspect of the invention, the antagonist is a peptide antagonist. More preferably, the peptide antagonist is an antibody or a fragment thereof, specific against NK1, NK2 and / or NK3 cell receptors, or combinations thereof. Even more preferably is an antibody which recognizes at least one sequence of NK1, NK2 or NK3 receptor, selected from: SEQ ID NO: 5, SEQ ID NO: 6 or SEQ ID NO: 7, or any fragment thereof.

The compositions of the present invention may be formulated for administration to an animal, more preferably a mammal, including a human being, in a variety of ways known in the state of the art. Hence they may be, without limitation, sterile aqueous or biological fluids such as saline solution. The aqueous solutions can be buffered or unbuffered and have additional active or inactive ingredients. Additional components include salts for modulating ionic strength, preservatives including, but not limited to, antimicrobials, antioxidants, chelating agents and similar, and nutrients including glucose, dextrose, vitamins and minerals. Alternatively, the compositions may be prepared for administration in solid form. The compositions may be combined with various vehicles or inert excipients, including but not limited to; binders such as microcrystalline cellulose, gum tragacanth, or gelatin; excipients such as starch or lactose; dispersing agents such as alginic acid or corn starch; lubricants such as magnesium stearate, glidants such as colloidal silicon dioxide; sweetening agents such as sucrose or saccharin; or flavoring agents such as peppermint or methyl salicylate.

Therefore, in a preferred embodiment of this aspect of the invention, the pharmaceutical composition may further comprise a pharmaceutically acceptable carrier. In another preferred embodiment the pharmaceutical composition further comprises excipients. In another preferred embodiment the pharmaceutical composition may comprise another active ingredient.

As used herein, the term "active ingredient", "active substance", "pharmaceutically active substance", "active ingredient" or "pharmaceutically active ingredient" means any component that potentially provides pharmacological activity or a different effect in the diagnosis, cure, mitigation, treatment, or prevention of disease, or affects the structure or function of the body of the human being or other animals. The term includes those components that promote a chemical change in the development of the medicine and are present in said medicine, in a projected modified form that provokes the specific activity or effect.

The term "drug" or "medicine", as used herein, refers to any substance used for prevention, diagnosis, alleviation, treatment or cure of diseases in humans and animals.

### KIT OR DEVICE OF THE INVENTION

Another aspect of the present invention relates to a kit or device hereinafter referred to as kit or device of the invention, comprising the necessary elements to analyze:
a) the level of NK1 receptor and / or
b) the level of the truncated form of the NK1 receptor and / or
c) the presence of mRNA which encodes NK1 receptor and/or
d) the presence of mRNA which encodes the truncated form of the NK1 receptor,
in the blood plasma of the biological sample obtained in step (a).

In a preferred embodiment the kit may contain oligonucleotides designed from a known sequence or mRNA from the genes, and / or capable of hybridizing with the mRNA sequence which encodes the NK1 receptor and the truncated form of the NK1 receptor, for subsequent PCR amplification.

Preferably, the kit or device of the invention comprises at least one antibody which is selected from:
a) a "full length" (complete form) anti-NK1 antibody or
b) an anti-NK1 antibody in its truncated form

In a preferred embodiment of this aspect of the invention, the antibody is human, humanized or synthetic. In another more preferred embodiment, the antibody is monoclonal. In another more preferred embodiment, the antibody is fluorochrome-labeled. More preferably the fluorochrome is selected from the list comprising Fluorescein (FITC), tetramethylrhodamine and derivatives, phycoerythrin (PE), PerCP, Cy5, Texas, allophycocyanin, or any combination thereof.

More preferably, the kit of the present invention comprises the means for comparing the amount detected in the different steps with a reference quantity.

The kit can further include, without any limitation, buffers, agents to prevent contamination, protein degradation inhibitors, etc. Furthermore, the kit can include all means and containers required for launch and optimization. Preferably, the kit further comprises instructions for carrying out any of the methods of the invention.

When the RQ-PCR technique for gene and / or protein quantifying (a technique for quantifying the sensitive and reproducible gene expression) is carried out using the kit of the invention, the kit should further comprise an oligonucleotide polyT primer as well as the oligonucleotide (s) of the kit. These reagents may optionally be included in the kit.

A Northern Blot technique involves the use of electrophoresis to separate the RNA samples by size and subsequent detection with an oligonucleotide(s) (hybridization probe) complementary to (part of) the target sequence of RNA of interest.

It is also possible that the oligonucleotide(s) are immobilized in spots on a (preferably solid) surface. In one of its embodiments, the kit comprises a microarray, or microarray of the invention. A RNA microarray is an array on a solid substrate (typically a glass slide or a cell in a thin silicon film) assessing large amounts of different RNA which are detectable by immobilized probes on specific spots on a solid substrate. Each spot contains a specific nucleic acid sequence, usually a DNA sequence like probes (or indicators). Although the number of spots is not limited in any way, there is a preferred embodiment wherein the microarray is customized for the methods of the invention. In one embodiment, said personalized matrix comprises fifty spots or less, such as thirty spots or less, including twenty spots or less. Therefore, another aspect of the invention relates to a microarray comprising oligonucleotides designed from a known sequence or the mRNA of the genes, and / or capable of hybridizing with the mRNA sequence of the truncated form of the NK1 receptor gene.

Another aspect of the invention relates to a microarray, henceforth microarray of the invention, comprising oligonucleotides or single channel microarrays designed from a known sequence or mRNA which encodes the NK1 receptor, and the short form (or truncated) of the NK1 receptor.

For example, oligonucleotides sequences are constructed on the surface of a chip by sequentially elongation of a growing chain with a single nucleotide using photolithography. Thus, oligonucleotides are anchored by the 3rd end using a method of selective activation of nucleotides, protected by a photolabile reagent, using selective incidence of light through a photomask. The photomask can be physical or virtual.

Thus, oligonucleotide probes may be made up of between 10 and 100 nucleotides, more preferably between 20 and 70 nucleotides, and even more preferably between 24 and 30 nucleóitidos. For gene expression quantification, approximately 40 oligonucleotides per gene are preferably used.

Synthesis *in situ* on a solid support (eg, glass) could be carried out by inkjet technology, requiring longer probes. The supports may be, without limitation, filters or NC (nitrocellulose) or nylon (loaded) membranes or silicon, or glass slides for microscopes coated with aminosilane, polylysine, aldehydes or epoxy. The probe is each one of the chip samples. The target is the simple to be analysed: messenger RNA, total RNA, a PCR fragment, etc.

Another aspect of the invention relates to a microarray protein, hereinafter protein microarray of the invention, comprising anti- NK1 and / or anti - NK1 receptor truncated antibodies. The probes are antibodies attached to glass slides and the blanks are saline solution or tissue.

Another aspect of the invention relates to a solid support, or protein chip, comprising at least one of the anti- and anti- antibodies or any combination thereof, to perform any of the methods of the invention.

The methods of the invention may include additional steps, such as separation of proteins by mono and bidimensional electrophoresis (2D-PAGE), or after prior digestion of a protein mixture (sample) with trypsin and then the purification and analysis of peptides by mass spectrometry (MS), such as MALDI-TOF or by multidimensional chromatography, using ICAT *(Isotope-coded affinity tags),* DIGE (*Differential gel electrophoresis*) or protein arrays.

Another aspect of the invention relates to a solid support, or DNA chip, comprising oligonucleotides or single channel microarrays designed from a known sequence or an mRNA of at least one of the genes which encode the NK1 receptor. Preferably it comprises oligonucleotides capable of detecting mRNA of all genes.

For example, the oligonucleotide sequences are constructed on the surface of the chip by sequential elongation of a growing chain with a single nucleotide using photolithography. Thus, oligonucleotides are anchored by the 3' end by a method of selective nucleotides activation, protected by a photolabile reagent, by selective incidence of light through a photomask. The photomask can be physical or virtual.

Thus, oligonucleotide probes may be between 10 and 100 nucleotides, more preferably between 20 and 70 nucleotides, and even more preferably between 24 and 30 nucleotides. For gene expression quantification, approximately 40 oligonucleotides per gene are preferably used.

Synthesis *in situ* on a solid support (eg, glass) could be carried out by inkjet technology, requiring longer probes. The supports may be, without limitation, filters or NC or nylon (loaded) membranes or silicon, or glass slides for microscopes coated with aminosilane, polylysine, aldehydes or epoxy. The probe is each one of the chip samples. The target is the sample to be analysed: messenger RNA, total RNA, a PCR fragment, etc.

Another aspect of the invention relates to a storage means which is readable by a computer program comprising instructions capable of causing a computer to perform the steps of any of the methods of the invention.

Another aspect of the invention relates to a transmissible signal comprising program instructions capable of causing a computer to perform the steps of any of the methods of the invention.

Another aspect of the invention relates to the use of the kit or device, the microarray, or microarray invention to carry out any of the methods of the invention.

The invention also extends to computer programs adapted so that any processing means can implement the methods of the invention. Such programs can take the form of source code, object code, an intermediate source code and object code, for example in partially compiled form, or in any other form suitable for use in the implementation of the processes according to the invention.

Computer programs also encompass 'cloud' applications based on said process.

Therefore, another **aspect** of the invention relates to a computer program comprising program instructions to cause a computer to carry out the process according to any of the methods of the invention.

In particular, the invention encompasses computer programs arranged on or inside a carrier. The carrier may be any entity or device capable of supporting the program. When the program is incorporated into a signal which may be conveyed directly by a cable or other device or means, the carrier may be constituted using said cable or other device or means. Alternatively, the carrier may be an integrated circuit in which the program is included, the integrated circuit being adapted to execute or to be used in executing the corresponding processes. For example, programs may be incorporoted into a storage medium such as a ROM, a CD ROM or a semiconductor ROM memory, a USB memory, or a magnetic recording medium, for example, a floppy disk or a hard disk. Alternatively, the programs may be supported on a transmissable carrier signal. For example, it could be an electrical or optical signal that could be transported via electrical or optical cable, by radio or by other means.

Therefore, another **aspect** of the invention relates to a storage medium which is readable by a computer program comprising instructions capable of causing a computer to perform the steps of any of the methods of the invention.

Another **aspect** of the invention relates to a transmissible signal comprising program instructions capable of causing a computer to perform the steps of any of the methods of the invention.

The terms "amino acid sequence", "peptide", "oligopeptide", "polypeptide" and "protein" are used interchangeably herein, and refer to a polymeric form of amino acids of any length, which can be coding or non-coding, chemically or biochemically modified.

In the present invention the term "variant" or "biologically active fragment" refers to those variants or fragments of the indicated peptides which have an equal physiological, metabolic or immunologic effect or have the same usefulness as those described. That is to say, they are functionally equivalent. These effects can be determined by conventional methods.

The term "identity", as used herein, refers to the proportion of identical nucleotides or amino acids between two nucleotide or amino acid sequences which are compared. Methods of sequence comparison are known in the state of the art, and include but are not limited to, the GAG program, including GAP (Devereux et al., Nucleic Acids Research 12: 287 (1984) Genetics Computer Group University of Wisconsin, Madison (WI); BLAST, BLASTN or BLASTP, and FASTA (Altschul et al. 1999. J. Mol Biol. 215: 403-410.

The terms "polynucleotide" and "nucleic acid" are used interchangeably herein, referring to polymeric forms of nucleotides of any length, either ribonucleotides (RNA) as deoxyribonucleotides (DNA). The terms "amino acid sequence", "peptide", "oligopeptide", "polypeptide" and "protein" are used interchangeably herein, and refer to a polymeric form of amino acids of any length, which can be coding or non-coding, chemically or biochemically modified.

Unless defined otherwise, all technical and scientific terms used herein have the same meaning commonly understood by the expert skilled in the field of the invention. Methods and similar or equivalent material to those described herein can effectively be used in the present invention. Throughout the description and claims the word "comprises" and its variants are not limitative and therefore not intended to exclude other technical features, additives, components or steps. On the contrary, the word "consists" and its variants do indicate limitations, referring only to the technical characteristics, additives, components or steps that accompany it. For the experts in the field, other objects, advantages and features of the invention will become apparent in part from the description and in part from the putting the invention into practice. The following examples and drawings are provided by way of illustration, and are not intended to be limiting for the present invention.

### EXAMPLES OF THE INVENTION

Next, examples are shown by way of illustration, not intended to be limiting for the present invention, which demonstrates the advantages of the invention.

### Example 1. Cancer patients show detectable levels of NK1 receptor and and the truncated form of the NK1 receptor in peripheral blood plasma. Levels of the truncated form of the NK1 receptor are elevated in the blood plasma of cancer patients.

To demonstrate that cancer patients have detectable levels of NK1 receptor and said truncated form of the receptor, blood plasma of healthy patients and blood plasma of cancer patients was taken. In all cases the presence of NK1 receptor and the truncated form of said receptor was determined by the ELISA method. To determine the level of the complete form of the NK1 receptor (hereinafter "value of the complete form of the NK1 receptor" or "VFC-NK1 R"), this was labeled with an antibody that binds to an epitope of the C-terminus of said receptor (sc-14116 Reference; Santacruz Biotechnology). This C-terminus is unique and specific to the complete form of the NK1 receptor. To determine the combined level of the complete form and the truncated form of the NK1 receptor (hereinafter "combined value of the complete and truncated forms of the NK1 receptor" or "VCFCT-NK1R") an antibody was used which binds to an epitope of the second extracellular receptor bridge (which is common to both complete and truncated forms of receptor) and, therefore, labels both the truncated form of the NK1 receptor and the complete form (or "full-length") of said receptor (reference sc-14115; Santacruz Biotechnology). To determine the level of the truncated form of the NK1 receptor, the proportion between the value of the combined value of both receptors and the level value of the complete form of the NK1 receptor (VCFCT-NK1R / VFC-NK1R) was found.

**Table 1. Ratio NK1 R VCFCT-and HRV-NK1 R (± standard deviation) in the plasma of the blood plasma of healthy human subjects and patients with various cancers. In brackets the number of human subjects studied is specified.**

| **Type of cancer (number of subjects studied).** | **Ratio VCFCT-NK1R / VFC-NK1R (± Standard Deviation)** | **Type of cancer (number of subjects studied)** | **Ratio VCFCT-NK1R / VFC-NK1R (± Standard Deviation)** |
|---|---|---|---|
| Healthy subjects (10) | 1 (± 0.01) | Bladder carcinoma (2) | 1.4 (± 0.1) |
| Gastric carcinoma (8) | 1.4 (± 0.1) | Malignant fibrous histiocytoma sarcoma (6) | 1.7 (± 0.3) |
| Colon carcinoma (7) | 1.6 (± 0.1) | Melanoma (4) | 1.6 (± 0.3) |
| Pancreatic carcinoma (6) | 1.7 (± 0.2) | Neuroblastoma (1) | 1.3 (± 0.1) |
| Renal cell carcinoma (8) | 1.8 (± 0.1) | B-cell leukemias (8) | 1.4 (± 0.2) |
| Breast carcinoma (12) | 1.5 (± 0.3) | T-cell leukemias (8) | 1.9 (± 0.3) |
| Ovarian carcinoma (8) | 1.9 (± 0.6) | Non-Hodgkin lymphomas, B-cell (6) | 1.8 (± 0.3) |
| Endometrial carcinoma (6) | 1.8 (± 0.5) | Non-Hodgkin lymphoma, T-cell (2) | 1.9 (± 0.2) |
| Uterine cervix carcinoma (4) | 1.7 (± 0.3) | Burkitt lymphoma (2) | 1.9 (± 0.4) |
| Non small-cell Lung carcinoma (7) | 1.5 (± 0.4) | Hodgkin lymphoma (8) | 1.8 (± 0.3) |
| Small-cell lung carcinoma (3) | 1.8 (± 0.2) | B-cell leukemias (6) | 1.9 (± 0.4) |
| Papillary thyroid carcinoma (4) | 1.4 (± 0.1) | T-cell leukemias (2) | 1.8 (± 0.3) |
| follicular thyroid carcinoma (4) | 1.6 (± 0.2) | Multiple Myeloma (2) | 1.8 (± 0.2) |

Table 1 shows the ratio between VCFCT-NK1R and VFC-NK1R (± standard deviation) is shown in healthy subjects and in patients with different types of cancer. It is shown that in healthy human subjects the ratio between the combined value of the full and truncated forms NK1 receptor (VCFCT-NK1R) and the value of the full form of the NK1 receptor (VFC-NK1R) is close to 1, which demonstrates that in the plasma of the blood plasma of healthy subjects the predominant (exclusive) form is the complete NK1 receptor. In cancer patients this ratio is greater than 1, demonstrating that in plasma from blood plasma of cancer patients the presence of a significant amount of the truncated form NK1 receptor is detectable.

### Example 2. Cancer patients show a ratio between the detectable level of NK1 receptor and the NK1 receptor in its truncated form, in peripheral blood plasma, which differs depending on the tumor stage.

In this example it is shown that cancer patients have a ratio between NK1 R VCFCT-and HRV-NK1 R which is greater in cases where the tumor is at a more advanced stage. To do this, in each case the VCFCT-NK1R and VFC-NK1R were determined by the method described in Example 1, For each type of cancer, patients in different developmental stages (being the least advanced stage I and stage III the most advanced) were selected. The stages were established according to the pathological and clinical criteria set forth by current clinical guidelines defined by the World Health Organization and scientific societies (Sobin LH, Gospodarowicz MK, Wittekind Ch. Eds. TNM Classification of Malignant Tumors, 7th ed . Wiley-Blackwell, Oxford 2009; url: http://www.uicc.org/resources/tnm ).

**Table 2. Ratio VCFCT-NK1R and VFC-NK1R (± standard deviation) in the plasma of the blood plasma of cancer patients depending on the stage of the cancer. In brackets the number of human subjects studied is specified.**

| **Type of cancer (number of subjects studied).** | **Ratio VCFCT-NK1 R / VFC-NK1 R (± standard deviation). tumor stage.** | | |
|---|---|---|---|
| | stage I | stage II | stage III |
| Gastric carcinoma (9) | 1.2 (± 0.1) | 1.5 (± 0.2) | 1.8 (± 0.2) |
| Colon carcinoma (6) | 1.3 (± 0.1) | 1.6 (± 0.2) | 1.9 (± 0.3) |
| Pancreatic carcinoma (6) | 1.4 (± 0.2) | 1.7 (± 0.3) | 1.9 (± 0.2) |
| Renal cell carcinoma (9) | 1.2 (± 0.1) | 1.5 (± 0.2) | 1.8 (± 0.2) |
| Breast carcinoma (9) | 1.3 (± 0.3) | 1.6 (± 0.2) | 1.8 (± 0.3) |
| Ovarian carcinoma (8) | 1.2 (± 0.6) | 1.5 (± 0.2) | 1.8 (± 0.2) |
| Endometrial carcinoma (7) | 1.4 (± 0.5) | 1.6 (± 0.3) | 1.7 (± 0.3) |
| Uterine cervix carcinoma (6) | 1.2 (± 0.3) | 1.5 (± 0.1) | 1.6 (± 0.3) |
| Lung carcinoma, non small-cell (8) | 1.4 (± 0.4) | 1.7 (± 0.2) | 1.7 (± 0.2) |
| Lung carcinoma, small-cell (9) | 1.3 (± 0.2) | 1.6 (± 0.3) | 1.8 (± 0.2) |
| Papillary thyroid carcinoma (6) | 1.2 (± 0.1) | 1.6 (± 0.3) | 1.9 (± 0.3) |
| Follicular thyroid carcinoma (6) | 1.3 (± 0.2) | 1.5 (± 0.2) | 1.7 (± 0.1) |
| Bladder cancer (8) | 1.2 (± 0.1) | 1.5 (± 0.2) | 1.8 (± 0.5) |
| Malignant fibrous histiocytoma sarcoma (8) | 1.2 (± 0.3) | 1.6 (± 0.1) | 1.9 (± 0.4) |
| Melanoma (6) | 1.3 (± 0.3) | 1.5 (± 0.2) | 1.8 (± 0.3) |
| B-cell leukemias (9) | 1.3 (± 0.2) | 1.6 (± 0.3) | 1.7 (± 0.3) |
| T-cell leukemias (9) | 1.3 (± 0.3) | 1.5 (± 0.2) | 1.8 (± 0.2) |
| Non-Hodgkin lymphomas, B-cell (8) | 1.2 (± 0.3) | 1.7 (± 0.2) | 1.8 (± 0.3) |
| Non-Hodgkin lymphoma, T-cell (7) | 1.2 (± 0.2) | 1.6 (± 0.1) | 1.9 (± 0.3) |
| Burkitt lymphoma (6) | 1.4 (± 0.4) | 1.7 (± 0.1) | 1.8 (± 0.2) |
| Hodgkin lymphoma (6) | 1.2 (± 0.3) | 1.5 (± 0.2) | 1.7 (± 0.3) |
| B-cell leukemias (6) | 1.4 (± 0.4) | 1.8 (± 0.2) | 1.8 (± 0.3) |
| T-cell leukemias (3) | 1.3 | 1.6 | 1.9 |
| Multiple Myeloma (3) | 1.2 | 1.5 | 1.9 |

### Example 3. Cancer patients show a ratio between the detectable level of NK1 receptor and the NK1 receptor in its truncated form, in peripheral blood plasma, which differs depending on the response to specific treatment for this disease.

In the present example it is shown that cancer patients have a ratio NK1 R VCFCT / HRV-NK1 R that is higher in cases where there is no response to treatment. Thus, the value of the ratio between NK1R VCFCT / HRV-NK1R decreases when there is a better response to treatment. To do this, in each case the NK1 R VCFCT-and HRV-NK1 R were determined using the method explained in Example 1.

**Table 3. Ratio between VCFCT-NK1R and VFC-NK1R (± standard deviation) in the plasma of the blood plasma of cancer patients based on the response to specific treatment. All cases were treated with standard chemotherapy and radiation patterns. None of the cases was an alternative for surgical treatment (the tumors were surgically ineradicable). In brackets the number of human subjects studied is specified.**

| **Type of cancer (number of subjects studied).** | **Ratio VCFCT-NK1 R / VFC-NK1 R (± standard deviation). Pre-treatment and posttreatment values (no response and response -NR- -R-).** | | |
|---|---|---|---|
| | Pretreatment | After treatment NR | After treatment R |
| Gastric carcinoma (6) | 1.2 (± 0.1) | 1.5 (± 0.2) | 1.8 (± 0.2) |
| Colon carcinoma (6) | 1.3 (± 0.1) | 1.6 (± 0.2) | 1.9 (± 0.3) |
| Pancreatic carcinoma (3) | 1.4 | 1.7 | 1.9 |
| Breast carcinoma (9) | 1.3 (± 0.3) | 1.6 (± 0.2) | 1.8 (± 0.3) |
| Ovarian carcinoma (8) | 1.2 (± 0.6) | 1.5 (± 0.2) | 1.8 (± 0.2) |
| Endometrial carcinoma (7) | 1.4 (± 0.5) | 1.6 (± 0.3) | 1.7 (± 0.3) |
| Non small-cell Lung carcinoma (8) | 1.4 (± 0.4) | 1.7 (± 0.2) | 1.7 (± 0.2) |
| Small-cell Lung carcinoma (9) | 1.3 (± 0.2) | 1.6 (± 0.3) | 1.8 (± 0.2) |
| Malignant fibrous histiocytoma sarcoma (8) | 1.2 (± 0.3) | 1.6 (± 0.1) | 1.9 (± 0.4) |
| Melanoma (6) | 1.3 (± 0.3) | 1.5 (± 0.2) | 1.8 (± 0.3) |
| B-cell leukemias (9) | 1.3 (± 0.2) | 1.6 (± 0.3) | 1.7 (± 0.3) |
| T-cell leukemias (9) | 1.3 (± 0.3) | 1.5 (± 0.2) | 1.8 (± 0.2) |
| Non-Hodgkin lymphomas (8) | 1.2 (± 0.3) | 1.7 (± 0.2) | 1.8 (± 0.3) |
| Leukemias (6) | 1.4 (± 0.4) | 1.8 (± 0.2) | 1.8 (± 0.3) |

### Example 4 Cancer patients show detectable levels of mRNA which encodes the NK1 receptor and the mRNA which encodes the truncated form of NK1 receptor in peripheral blood plasma. Levels of the mRNA which encodes the truncated form of the NK1 receptor are elevated in the blood plasma of cancer patients.

To demonstrate that cancer patients have detectable levels of mRNA which encodes the NK1 receptor and mRNA which encodes the truncated form of said receptor, blood plasma of healthy patients and blood plasma of cancer patients was taken. In all cases, the presence of mRNA which encodes the NK1 receptor and the mRNA which encodes the truncated form of the receptor, was determined by the PCR method in said plasma using probes ("primers") with the characteristics that are detailed in table 4. In order to determine the mRNA level which encodes the complete form of the NK1 receptor (hereinafter "value of the mRNA which encodes the complete form of the NK1 receptor" or "VARNmFC-NK1R") the primer (described in table 4 under the reference "fl-TACR1") was used. To determine the level of mRNA which encodes the truncated form of the NK1 receptor (hereinafter "value of the mRNA which encodes the truncated form of NK1 receptor" or "VARNmFT-NK1 R") a primer (whose characteristics are shown in table 4 under the reference "tr-TACR1") was used that binds to a sequence which is common to both long and truncated mRNA which encodes both forms of the receptor.

**Table 4. Characteristics of the probes used for detection of full and truncated forms of the NK1 receptor.**

| | **transcribed** | **Reference Sequence** | **Catalogue Number** | **Amplification Size** | **Position** |
|---|---|---|---|---|---|
| **Complete form of the NK1 receptor.** | fl-TACR1 (Full length TACR1) | NM_001058 | PPH68645A | 58 | 4264-4285 |
| **Short or truncated form of the NK1 receptor.** | tr-TACR1 (trucanted TACR1) | NM_015727 | PPH68646A | 80 | 1581-1604 |

**Table 5. Ratio VARNmFT-NK1R / VARNmFC-NK1R (± standard deviation) in the plasma of the blood plasma of healthy human subjects and patients with various types of cancer. In brackets the number of human subjects studied is specified.**

| malignant fibrous histiocytoma | malignant fibrous histiocytoma | malignant fibrous histiocytoma | malignant fibrous histiocytoma |
|---|---|---|---|
| Healthy subjects (10) | 0 (± 0.04) | Bladder carcinoma (2) | 2.3 (± 0.2) |
| Gastric carcinoma (8) | 0.6 (± 0.2) | Malignant fibrous histiocytoma sarcoma (6) | 2.1 (± 0.2) |
| Colon carcinoma (7) | 1.3 (± 0.2) | Melanoma (4) | 1.9 (± 0.2) |
| Pancreatic carcinoma (6) | 1.5 (± 0.5) | Neuroblastoma (1) | 1.4 (± 0.3) |
| Renal cell carcinoma (8) | 1.2 (± 0.3) | B-cell leukemias (8) | 3.1 (± 0.4) |
| Breast carcinoma (12) | 1.6 (± 0.4) | T-cell leukemias (8) | 2.4 (± 0.2) |
| Ovarian carcinoma (8) | 1.7 (± 0.3) | Non-Hodgkin lymphomas, B-cell (6) | 2.2 (± 0.3) |
| Endometrial carcinoma (6) | 1.9 (± 0.3) | Non-Hodgkin lymphoma, T-cell (2) | 3.1 (± 0.4) |
| Uterine cervix carcinoma (4) | 1.9 (± 0.2) | Burkitt lymphoma (2) | 2.8 (± 0.5) |
| Non small-cell Lung carcinoma (7) | 2.1 (± 0.3) | Hodgkin lymphoma (8) | 2.5 (± 0.4) |
| Small lung carcinoma cells (3) | 1.9 (± 0.1) | B-cell leukemias (6) | 2.3 (± 0.6) |
| Papillary thyroid carcinoma (4) | 2.2 (± 0.6) | T-cell leukemias (2) | 2.5 (± 0.4) |
| Follicular thyroid carcinoma (4) | 1.8 (± 0.3) | Multiple Myeloma (2) | 2.4 (± 0.5) |

Table 5 shows the ratio between VARNmFT-NK1R / VARNmFC-NK1R (± standard deviation) in healthy subjects and in patients with different types of cáncer. It is shown that in healthy human subjects the ratio between the combined value of the mRNA of the complete and truncated forms of the NK1 receptor (VARNmFT-NK1R / VARNmFC-NK1R) is close to 0, which shows that in the blood plasma of the healthy subjects, the predominant (exclusive) form is the mRNA which encodes the complete form of the NK1 receptor. In cancer patients this ratio is greater than 0, even exceeding 1 or 2, which shows that in the plasma of the blood plasma of patients with cancer, the presence of a significant amount of mRNA of the truncated form of NK1 receptor is detectable.

### Example 5. Cancer patients show a ratio between detectable levels of mRNA which encodes the NK1 receptor and the mRNA which encodes the truncated form of the NK1 receptor in the plasma of peripheral blood which differs depending on the stage of the tumor.

In this example it is shown that cancer patients have a ratio between VARNmFT-NK1R and VARNmFC-NK1R which is greater in cases where the tumor is at a more advanced stage. To do this, in each case the VARNmFT-NK1R / VARNmFC-NK1R was determined by the method described in Example 4. For each type of cancer, patients in different developmental stages (being the least advanced stage I and stage III the most advanced) were selected. The stages were established according to the pathological and clinical criteria set forth by current clinical guidelines defined by the World Health Organization and scientific societies (Sobin LH, Gospodarowicz MK, Wittekind Ch. Eds. TNM Classification of Malignant Tumors, 7th ed . Wiley-Blackwell, Oxford 2009; url: http://www.uicc.org/resources/tnm ).

**Table 6. Ratio VARNmFT-NK1R / VARNmFC-NK1R (± standard deviation) in the plasma of the blood plasma of cancer patients depending on the stage of the cancer. In brackets the number of human subjects studied is specified. It is demonstrated that the presence of mRNA which encodes the truncated form of NK1 receptor detectable in the blood plasma of cancer patients is higher in patients with more advanced cancers (more advanced tumor stage).**

| **Type of cancer (number of subjects studied).** | **Ratio VARNmFT-NK1 R / VARNmFC-NK1 R (± standard deviation). Tumor stage.** | | |
|---|---|---|---|
| | **stage I** | **stage II** | **stage III** |
| Gastric carcinoma (9) | 0.4 (± 0.1) | 1.9 (± 0.3) | 2.5 (± 0.7) |
| Colon carcinoma (6) | 0.6 (± 0.1) | 1.9 (± 0.3) | 2.5 (± 0.6) |
| Pancreatic carcinoma (6) | 0.6 (± 0.2) | 1.5 (± 0.2) | 2.4 (± 0.5) |
| Renal cell carcinoma (9) | 0.7 (± 0.1) | 1.5 (± 0.3) | 1.9 (± 0.4) |
| Breast carcinoma (9) | 0.5 (± 0.3) | 1.3 (± 0.4) | 1.9 (± 0.5) |
| Ovarian carcinoma (8) | 0.6 (± 0.6) | 1.4 (± 0.3) | 1.9 (± 0.5) |
| Endometrial carcinoma (7) | 0.6 (± 0.5) | 1.5 (± 0.2) | 2.8 (± 0.4) |
| Uterine cervix carcinoma (6) | 0.7 (± 0.3) | 1.2 (± 0.3) | 2.1 (± 0.6) |
| Non small-cell Lung carcinoma, (8) | 0.6 (± 0.4) | 1.7 (± 0.2) | 2.9 (± 0.5) |
| Small-cell Lung carcinoma, (9) | 0.5 (± 0.2) | 1.4 (± 0.2) | 3.2 (± 0.4) |
| Papillary thyroid carcinoma (6) | 0.7 (± 0.1) | 1.5 (± 0.4) | 2.5 (± 0.5) |
| Follicular thyroid carcinoma (6) | 0.6 (± 0.2) | 1.6 (± 0.3) | 2.1 (± 0.4) |
| Bladder cancer (8) | 0.7 (± 0.1) | 1.7 (± 0.4) | 2.5 (± 0.2) |
| Malignant fibrous histiocytoma sarcoma (8) | 0.6 (± 0.3) | 1.7 (± 0.3) | 2.9 (± 0.3) |
| Melanoma (6) | 0.6 (± 0.3) | 1.7 (± 0.3) | 3.6 (± 0.4) |
| B-cell leukemias (9) | 1.2 (± 0.2) | 2.2 (± 0.2) | 3.9 (± 0.5) |
| T-cell leukemias (9) | 1.3 (± 0.3) | 2.3 (± 0.5) | 3.8 (± 0.4) |
| Non-Hodgkin lymphomas, B-cell (8) | 1.5 (± 0.3) | 2.4 (± 0.4) | 3.8 (± 0.5) |
| Non-Hodgkin lymphoma, T-cell (7) | 1.6 (± 0.2) | 2.3 (± 0.4) | 3.9 (± 0.4) |
| Burkitt lymphoma (6) | 1.8 (± 0.4) | 2.4 (± 0.4) | 3.6 (± 0.5) |
| Hodgkin lymphoma (6) | 1.2 (± 0.3) | 2.4 (± 0.5) | 2.9 (± 0.4) |
| B-cell leukemias (6) | 1.9 (± 0.4) | 2.4 (± 0.4) | 3.6 (± 0.5) |
| T-cell leukemias (3) | 1.9 | 2.5 | 3.8 |

### Example 6. Cancer patients have a ratio between the detectable level of NK1 receptor and the truncated form of the NK1 receptor, in peripheral blood plasma, which differs depending on the response to specific treatment for this disease.

In the present example it shows that cancer patients have a ratio VARNmFT-NK1R / VARNmFC-NK1 R that is higher in cases where there is no response to treatment. Thus, the value of the ratio VARNmFT-NK1R / VARNmFC-NK1R is decreases when the response to treatment is better. To do this, in each case VARNmFT-NK1R and VARNmFC-NK1R were determined by the method explained in Example 4.

**Table 7. Ratio VARNmFT-NK1R / VARNmFC-NK1R (± standard deviation) in the plasma of the blood plasma of cancer patients based on the response to specific treatment chemotherapy and / or radiotherapy. All cases were exclusively subsidiary of chemotherapy treatment and / or radiotherapy without (not surgically eradicable) surgical options. In brackets the number of human subjects studied is specified.**

| **Type of cancer (number of subjects studied).** | **Ratio VARNmFT-NK1 R / VARNmFC-NK1 R** (± **standard deviation). Pre-treatment and posttreatment values (no response and response -NR- -R-).** | | |
|---|---|---|---|
| | **Pre-treatment** | **After treatment NR** | **After treatment R** |
| Gastric carcinoma (6) | 2.9 (± 0.2) | 3.6 (± 0.3) | 0.2 (± 0.1) |
| Colon carcinoma (6) | 1.9 (± 0.2) | 3.2 (± 0.3) | 0.5 (± 0.1) |
| Pancreatic carcinoma (3) | 2.5 | 3.5 | 0.7 |
| Breast carcinoma (9) | 2.6 (± 0.4) | 3.5 (± 0.3) | 0.7 (± 0.1) |
| Ovarian carcinoma (8) | 1.9 (± 0.5) | 2.6 (± 0.4) | 0.8 (± 0.3) |
| Endometrial carcinoma (7) | 2.6 (± 0.6) | 3.7 (± 0.4) | 0.9 (± 0.2) |
| Non small-cell Lung carcinoma (8) | 1.9 (± 0.5) | 2.5 (± 0.5) | 0.9 (± 0.3) |
| Small-cell Lung carcinoma, (9) | 3.2 (± 0.5) | 4.3 (± 0.4) | 0.7 (± 0.1) |
| Maligno- -histiocitoma fibrous sarcoma (8) | 2.5 (± 0.3) | 3.1 (± 0.3) | 0.8 (± 0.2) |
| Melanoma (6) | 1.9 (± 0.4) | 2.2 (± 0.4) | 0.6 (± 0.2) |
| B-cell leukemias (9) | 4.1 (± 0.4) | 5.3 (± 0.3) | 0.5 (± 0.1) |
| T-cell leukemias (9) | 3.6 (± 0.5) | 4.2 (± 0.3) | 0.6 (± 0.2) |
| Non-Hodgkin lymphomas (8) | 3.7 (± 0.5) | 4.4 (± 0.4) | 0.5 (± 0.2) |
| Leukemias (6) | 3.6 (± 0.5) | 4.2 (± 0.5) | 0.7 (± 0.3) |

### BIBLIOGRAPHY

- Adams GP, Weiner LM. Monoclonal antibody therapy of cancer. Nat Biotechnol. 2005 Sep;23(9):1147-57.
- Barker R. Tachykinins, neurotrophism and neurodegenerative diseases: a critical review on the possible role of tachykinins in the aetiology of CNS diseases. Neurosci. Res., 1996, 7, 187-214.
- Bigioni M, Benzo A, Irrissuto C, Maggi CA, Goso C. Role of NK-1 and NK-2 tachykinin receptor antagonism on the growth of human breast carcinoma cell line MDA-MB-231. Anticancer Drugs. 2005,16(10):1083-9.
- Bunn PA Jr, Chan D, Stewart J, Gera L, Tolley R, Jewett P, Tagawa M, Alford C, Mochzuki T, Yanaihara N. Effects of neuropeptide analogues on calcium flux and proliferation in lung cancer cell lines. Cancer Res. 1994;54(13):3602-10.
- De Bari C, Dell'Accio F, Tylzanowski P, Luyten FP. Multipotent mesenchymal stem cells from adult human synovial membrane. Arthritis Rheum. 2001 ;44(8): 1928-42.
- Di Fiore F, Blanchard F, Charbonnier F, Le Pessot F, Lamy A, Galais MP, Bastit L, Killian A, Sesboüé R, Tuech JJ, Queuniet AM, Paillot B, Sabourin JC, Michot F, Michel P, Frebourg T. Clinical relevance of KRAS mutation detection in metastatic colorectal cancer treated by Cetuximab plus chemotherapy. Br J Cancer. 2007 Apr 23;96(8):1166-9
- Doi T, Kamo I, Imai S, Okanishi S, Ishimaru T, Ikeura Y, Natsugari H. Effects of TAK- 637, a tachykinin receptor antagonist, on lower urinary tract function in the guinea pig. Eur J Pharmacol. 1999;383(3):297-303.
- Giardina GA, Gagliardi S, Martinelli M. Antagonists at the neurokinin receptors-Recent patent literature. IDrugs. 2003; 6(8):758-72.
- Gillespie E, Leeman SE, Watts LA, Coukos JA, O'Brien MJ, Cerda SR, Farraye FA, Stucchi AF, Becker JM. Truncated neurokinin-1 receptor is increased in colonic epithelial cells from patients with colitis-associated cancer. Proc Natl Acad Sci U S A. 2011 Oct 18;108(42):17420-5.
- Huang SC, Korlipara VL. Neurokinin-1 receptor antagonists: a comprehensive patent survey. Expert Opin Ther Pat. 2010 Aug;20(8):1019-45. Review).
- Kage R, Leeman SE, Boyd ND. Biochemical characterization of two different forms of the substance P receptor in rat submaxillary gland. J Neurochem. 1993; 60:347-351.
- Kramer MS, Cutler N, Feighner J, Shrivastava R, Carman J, Sramek JJ, Reines SA, Liu G, Snavely D, Wyatt-Knowles E, Hale JJ, Mills SG, MacCoss M, Swain CJ, Harrison T, Hill RG, Hefti F, Scolnick EM, Cascieri MA, Chicchi GG, Sadowski S, Williams AR, Hewson L, Smith D, Carlson EJ, Hargreaves RJ, Rupniak NM. Distinct mechanism for antidepressant activity by blockade of central substance P receptors. Science. 1998. 11;281 (5383):1640-5.
- Maggi CA, Patacchini R, Rovero R, Giachetti A. Tachykinin Receptor and Tachykinin Receptor Antagonist. Journal of Autonomic Pharmacology. 1993 (13):23-93.
- Muñoz M, González-Ortega A, Rosso M, Robles-Frias MJ, Carranza A, Salinas-Martín MV, Coveñas R. The substance P/neurokinin-1 receptor system in lung cancer: Focus on the antitumor action of neurokinin-1 receptor antagonists. Peptides. 2012 Dec;38(2):318-25.
- Muñoz M, Rosso M, Robles-Frias MJ, Salinas-Martín MV, Rosso R, González-Ortega A, Coveñas R. The NK-1 receptor is expressed in human melanoma and is involved in the antitumor action of the NK-1 receptor antagonist Aprepitant on melanoma cell lines. Lab Invest. 2010; 90(8):1259-69
- Orosz A, Schrett J, Nagy J, Bartha L, Schön I, Nyéki O. New short-chain analogs of a substance-P antagonist inhibit proliferation of human small-cell lung-cancer cells in vitro and in vivo. Int J Cancer. 1995;60(1):82-7.
- Palma C, Maggi CA. The role of tachykinins via NK1 receptors in progression of human gliomas. Life Sci. 2000;67(9):985-1001.
- Quartara L, Maggi CA. The tachykinin NK1 receptor. Part II: Distribution and pathophysiological roles. Neuropeptides. 1998;32(1):1-49.
- Rosso M, Robles-Frías MJ, Coveñas R, Salinas-Martín MV, Muñoz M. The NK-1 receptor is expressed in human primary gastric and colon adenocarcinomas and is involved in the antitumor action of L-733,060 and the mitogenic action of substance P on human gastrointestinal cancer cell lines. Tumour Biol. 2008;29(4):245-54.
- Singh D, Joshi DD, Hameed M, Qian J, Gascón P, Maloof PB, Mosenthal A, Rameshwar P. Increased expression of preprotachykinin-I and neurokinin receptors in human breast cancer cells: implications for bone marrow metastasis. Proc Natl Acad Sci USA. 2000;97(1):388-93.
- Sobin LH, Gospodarowicz MK, Wittekind Ch. Eds. TNM Classification of Malignant Tumors, 7th ed. Wiley-Blackwell, Oxford 2009.

## Claims

1. The use of the short (or truncated) form of the NK1 receptor or mRNA that encodes it to:
a) Diagnose cancer in an individual,
b) Monitor the progress of cancer in an individual,
c) Classify an individual into a stage of disease,
d) Predict response of said individual to cancer treatment.

2. The use according to the earlier claim in which the diagnosis is an early diagnosis.

3. The use according to any of claims 1, wherein the treatment response is predicted using a NK1 receptor antagonist.

4. An *in vitro* method of obtaining useful data for diagnosing cancer in an individual, comprising detecting expression levels of the short (or truncated) form of the NK1 receptor, or the mRNA which encodes it, in an isolated biological sample from said individual.

5. An *in vitro* method for diagnosing cancer in an individual comprising:
a) obtaining an isolated biological sample from an individual,
b) detecting the expression levels of the short or truncated form NK1 receptor, or mRNA which encodes it, in the biological sample of step (a), and
c) assigning that individual to the group of individuals suffering from cancer when the short or truncated form NK1 receptor or its mRNA expression exhibits a superior level relative to a reference sample.

6. The method according to the preceding claim wherein superior expression or overexpression is defined as a level of expression of the truncated form of the NK1 receptor, or mRNA which encodes the truncated form of the NK1 receptor compared to the level of expression of the complete form of the NK1 receptor, or mRNA which encodes the complete form of the NK1 receptor, respectively, increased by at least more than:
a) 0.01%
b) 0.1%
c) 1%
d) 10%
e) 20%
f) 30%
g) 40%
h) 50%
i) 60%
j) 70%, or
k) 80%
in the isolated biological sample from the individual.

7. An *in vitro* method for diagnosing cancer in an individual comprising:
a) obtaining an isolated biological sample from an individual,
b) simultaneously detecting expression levels of the NK1 receptor and its short or truncated form, or mRNA that encodes it, in the biological sample of step (a), and
c) assigning that individual to the group of individuals suffering from cancer when the ratio between the expression levels of NK1 receptor and expression levels of its truncated form, or the ratio between mRNA which encodes them respectively, has a value greater than 1.

8. An in vitro method for classifying an individual in a particular tumor stage comprising the steps (a) - (c) according to any of claims 5-7, and further comprising classifying the individual in the group of individuals suffering from cancer:
i) stage I, when the value of the ratio between the sum of the expression levels of the complete form and the truncated form of the NK1 receptor and the level of expression of the complete form of said receptor, is greater than 1 and less than 1.5 and / or the value of the ratio between levels of mRNAs which encode the truncated and the complete form of the receptor is greater than 0 and less than 1.2;
ii) Stage II, when the value of the ratio between the sum of the expression levels of the complete form and the truncated form of the NK1 receptor and the level of expression of the complete form of said receptor is greater than 1.5 and less than 1.6 and / or the value of the ratio between levels of mRNAs which encode the complete form and the truncated form of the receptor is greater than 1.2 and less than 1.9;
iii) stage III, when the value of the ratio between the sum of the levels of the complete form and truncated form of the NK1 receptor and the level of the complete form of said receptor is greater than 1.6 and less than 1, 9 and / or the value of the ratio between levels of mRNAs which encode the truncated and complete form of receptor is greater than 1.9 and less than 3.9;
iv) Stage IV, when the value of the ratio between the sum of the levels of the entire shape and truncated NK1 receptor and the level of the entire form of said receptor is greater than 1.9 and / or value mRNA levels which encodes the truncated complete form of the receptor is greater than 3.9.

9. An in vitro method to evaluate the evolution of an individual diagnosed with cancer comprising detecting expression levels of the NK1 receptor and / or its short form (or truncated) or mRNA that encodes it, in a biological sample from said individual, at two or more different times.

10. The method of any of claims 3-9, wherein the biological sample is peripheral blood plasma obtained from an individual.

11. The method of any of claims 3-10, wherein the biological sample is blood plasma obtained from an individual.

12. The method of any of claims 3-11, wherein the detection of
a. expression levels of the complete form of the receptor NK1
b. expression levels of the truncated form of the NK1 receptor
c. the presence of the mRNA which encodes the complete form of the NK1 receptor.
d. the presence of the mRNA which encodes the complete form of the NK1 receptor.
It is performed by
i. a process of genetic profiling, such as a microarray, and / or
ii. a method comprising PCR such as real-time PCR; and/or
iii. Northern blotting, and / or
iv. Western blotting and / or
v. an immunohistochemical procedure and / or
vi. an ELISA method.

13. The method of any of claims 3-12, wherein detection of the expression levels is performed using Q-RT-PCR.

14. The method of any of claims 3-13, wherein the detection of protein levels of the complete form of the NK1 receptor and / or the truncated form of the NK1 receptor is performed using immunological techniques.

15. The method of claim 14 wherein immunological techniques are based on precipitation reactions, agglutination based reactions, immunostaining, and radioimmunoassay and radioimmunoradiometric techniques, ELISA method, or any combination thereof.

16. The method of any of claims 14 to 15, where immunological techniques include immunostaining.

17. The method of claim 16, wherein the immunolabeling is selected from immunostataining with antibodies conjugated with enzymes, immunostainig with antibodies conjugated with fluorohrome or cytometry.

18. The method of any of claims 4-17, wherein the cancer is selected from the following: gastric cancer, gastric adenocarcinoma preferably; colon carcinoma, preferably colon adenocarcinoma; pancreatic cancer, preferably pancreatic adenocarcinoma; renal cancer, preferably renal clear cell carcinoma; breast cancer, breast adenocarcinoma preferably; ovarian carcinoma, ovarian adenocarcinoma preferably; endometrial cancer; uterine cervical cancer; lung cancer, preferably non small-cell lung carcinoma and / or small-cell lung carcinoma; thyroid cancer, papillary preferably thyroid carcinoma and / or follicular thyroid carcinoma; bladder cancer, preferably transitional cell carcinoma of urinary bladder; prostate carcinoma; glial lineages cancer of the Central Nervous System or glioma; sarcomas, preferably fibrosarcoma, malignant fibrous histiocytoma and Ewing sarcoma; melanoma; embryonal cancers, preferably neuroblastoma; and hematologic cancers, preferably B-cell or T-cell leukemia, non-Hodgkin lymphomas, preferably B-cell or T-cell, Burkitt's lymphoma, Hodgkin's lymphoma and multiple myeloma.

19. A pharmaceutical composition comprising an antagonist NK1 receptor in the preparation of a medicine for the treatment of an individual identified by a method as described in any of claims 4-18, as an individual suffering from a cancer, or who can be classified as being in a particular stage of cancer.

20. The composition of claim 19, wherein the antagonist NK1 receptor is a non-peptide antagonist which is selected from the list consisting of: aprepitant, Vestipitant, Casopitant, Vofopitant, Ezlopitant, lanepitant, LY-686017, L-733,060 , L-732, 138, L-703.606, WIN 62.577, CP-122721, TAK-637, and R673, CP-100263, WIN 51708, CP-96345, L-760735, CP-122721, L-758298, L -741 671, L-742694, CP-99994, T-2328, or any combination thereof.

21. The composition of claim 19, wherein the NK1 receptor antagonist is an antibody or a fragment thereof, specific against NK1, NK2 and / or NK3 cell receptors, or combinations thereof, which recognizes at least one sequence NK1, NK2 or NK3 receptor, selected from: SEQ ID NO: 5, SEQ ID NO: 6 or SEQ ID NO: 7.

22. A kit or device comprising primers, probes and / or antibodies capable of detecting at least the truncated form of NK1 receptor or mRNA which encodes it, and preferably also comprising primers, probes and / or antibodies capable of detecting the complete form of the NK1 receptor, or the mRNA which encodes it and where:
- Primers are sequences of polynucleotides of between 10 and 30 debases pairs, more preferably between 15 and 25 base pairs, even more preferably between 18 and 22 base pairs, and even more preferably from about 20 pairs of bases, which have an identity of at least 80%, more preferably at least 90%, even more preferably at least 95%, even more preferably at least 98% and particularly 100%, with a fragment of the complementary sequence of SEQ ID NO: 4 (short form of the NK1 receptor) and / or SEQ ID NO: 2 (long form of the NK1 receptor)
- The probes are polynucleotide sequences of between 80 and 1100 base pairs, more preferably between 100 and 1000 base pairs, and most preferably between 200 and 500 base pairs, which have an identity of at least 80% , more preferably at least 90%, even more preferably at least 95%, even more preferably at least 98%, particularly 100%, with a fragment of the complementary sequences to SEQ ID NO : 4 (short form of the NK1 receptor) and / or SEQ ID NO: 2 (long form of the NK1 receptor),
- The antibodies are capable of binding specifically to a region formed by any of the amino acid sequences SEQ ID NO: 1 (long form of the NK1 receptor), SEQ ID NO: 3 (short form of the NK1 receptor)

23. The kit or device according to claim 22, comprising at least one antibody which is selected from:
a) an anti-NK1 antibody in its complete form.
b) an anti-NK1 antibody in its truncated form.

24. The kit or device according to claim 23, wherein the anti-NK1 antibody in its complete form recognizes an epitope located at the C-terminus receptor.

25. The kit or device according to claim 23, wherein the anti-NK1 in its truncated form antibody recognizes an epitope on one of the transmembrane bridges of the receptor.

26. The kit or device according to any of claims 22-25, wherein the antibody is monoclonal.

27. The kit or device according to any of claims 22-26, wherein the antibody is labeled with a fluorochrome.

28. The kit or device according to claim 27, wherein the fluorochrome is selected from the list comprising: Fluorescein (FITC), tetramethylrhodamine and derivatives, phycoerythrin (PE), PerCP, Cy5, Texas, allophycocyanin, or any combination thereof.

29. Use of a kit or device according to any of claims 22 to 28 to carry out a method as described in any of claims 4 to 18.


SEQUENCE LISTING

<110>  SERVICIO ANDALUZ DE SALUD

<120>  Método para diagnosticar y monitorizar la presencia de cáncer en
       un sujeto humano.

<130>  P12048PC00

<160>  7

<170>  PatentIn version 3.5

<210>  1
<211>  407
<212>  PRT
<213>  Homo sapiens

<400>  1

Met Asp Asn Val Leu Pro Val Asp Ser Asp Leu Ser Pro Asn Ile Ser
1               5                   10                  15

Thr Asn Thr Ser Glu Pro Asn Gln Phe Val Gln Pro Ala Trp Gln Ile
            20                  25                  30

Val Leu Trp Ala Ala Ala Tyr Thr Val Ile Val Val Thr Ser Val Val
        35                  40                  45

Gly Asn Val Val Val Met Trp Ile Ile Leu Ala His Lys Arg Met Arg
    50                  55                  60

Thr Val Thr Asn Tyr Phe Leu Val Asn Leu Ala Phe Ala Glu Ala Ser
65                  70                  75                  80

Met Ala Ala Phe Asn Thr Val Val Asn Phe Thr Tyr Ala Val His Asn
                85                  90                  95

Glu Trp Tyr Tyr Gly Leu Phe Tyr Cys Lys Phe His Asn Phe Phe Pro
            100                 105                 110

Ile Ala Ala Val Phe Ala Ser Ile Tyr Ser Met Thr Ala Val Ala Phe
        115                 120                 125

Asp Arg Tyr Met Ala Ile Ile His Pro Leu Gln Pro Arg Leu Ser Ala
    130                 135                 140

Thr Ala Thr Lys Val Val Ile Cys Val Ile Trp Val Leu Ala Leu Leu
145                 150                 155                 160

Leu Ala Phe Pro Gln Gly Tyr Tyr Ser Thr Thr Glu Thr Met Pro Ser
                165                 170                 175

Arg Val Val Cys Met Ile Glu Trp Pro Glu His Pro Asn Lys Ile Tyr
                180                 185                 190

Glu Lys Val Tyr His Ile Cys Val Thr Val Leu Ile Tyr Phe Leu Pro
            195                 200                 205

Leu Leu Val Ile Gly Tyr Ala Tyr Thr Val Val Gly Ile Thr Leu Trp
    210                 215                 220

Ala Ser Glu Ile Pro Gly Asp Ser Ser Asp Arg Tyr His Glu Gln Val
225                 230                 235                 240

Ser Ala Lys Arg Lys Val Val Lys Met Met Ile Val Val Val Cys Thr
                245                 250                 255

Phe Ala Ile Cys Trp Leu Pro Phe His Ile Phe Phe Leu Leu Pro Tyr
                260                 265                 270

Ile Asn Pro Asp Leu Tyr Leu Lys Lys Phe Ile Gln Gln Val Tyr Leu
            275                 280                 285

Ala Ile Met Trp Leu Ala Met Ser Ser Thr Met Tyr Asn Pro Ile Ile
    290                 295                 300

Tyr Cys Cys Leu Asn Asp Arg Phe Arg Leu Gly Phe Lys His Ala Phe
305                 310                 315                 320

Arg Cys Cys Pro Phe Ile Ser Ala Gly Asp Tyr Glu Gly Leu Glu Met
                325                 330                 335

Lys Ser Thr Arg Tyr Leu Gln Thr Gln Gly Ser Val Tyr Lys Val Ser
                340                 345                 350

Arg Leu Glu Thr Thr Ile Ser Thr Val Val Gly Ala His Glu Glu Glu
            355                 360                 365

Pro Glu Asp Gly Pro Lys Ala Thr Pro Ser Ser Leu Asp Leu Thr Ser
    370                 375                 380

Asn Cys Ser Ser Arg Ser Asp Ser Lys Thr Met Thr Glu Ser Phe Ser
385                 390                 395                 400

Phe Ser Ser Asn Val Leu Ser
                405

<210>  2
<211>  4795

<212>  DNA
<213>  Homo sapiens

<400>  2
agctgagcaa cccgaaccga gaggtgcccg cgaaactgca ggcggcggca gcggcagcaa      60

aagagaagga aaaatctcca gctggatacg aagctccaga atcctggcca taggctcaga     120

acttttacag gtcgcgctgc aatgggcccc cacttcgctc ctaagtcctc acgcagcaca     180

gggctttgcc tttccctgcg gaggaaggag aaataggagt tgcaggcagc agcaggtgca     240

taaatgcggg ggatctcttg cttcctagaa ctgtgaccgg tggaatttct ttcccttttt      300

cagtttaccg caagagagat gctgtctcca gacttctgaa ctcaaacgtc tcctgaagct     360

tgaaagtgga ggaattcaga gccaccgcgg gcaggcgggc agtgcatcca gaagcgttta     420

tattctgagc gccagttcag ctttcaaaaa gagtgctgcc cagaaaaagc cttccaccct    480

cctgtctggc tttagaagga ccctgagccc caggcgccag ccacaggact ctgctgcaga     540

ggggggttgt gtacagatag tagggcttta ccgcctagct tcgaaatgga taacgtcctc    600

ccggtggact cagacctctc cccaaacatc tccactaaca cctcggaacc caatcagttc     660

gtgcaaccag cctggcaaat tgtcctttgg gcagctgcct acacggtcat tgtggtgacc     720

tctgtggtgg gcaacgtggt agtgatgtgg atcatcttag cccacaaaag aatgaggaca     780

gtgacgaact attttctggt gaacctggcc ttcgcggagg cctccatggc tgcattcaat     840

acagtggtga acttcaccta tgctgtccac aacgaatggt actacggcct gttctactgc     900

aagttccaca acttctttcc catcgccgct gtcttcgcca gtatctactc catgacggct     960

gtggcctttg ataggtacat ggccatcata catcccctcc agccccggct gtcagccaca    1020

gccaccaaag tggtcatctg tgtcatctgg gtcctggctc tcctgctggc cttcccccag    1080

ggctactact caaccacaga gaccatgccc agcagagtcg tgtgcatgat cgaatggcca    1140

gagcatccga acaagattta tgagaaagtg taccacatct gtgtgactgt gctgatctac    1200

ttcctccccc tgctggtgat tggctatgca tacaccgtag tgggaatcac actatgggcc    1260

agtgagatcc ccggggactc ctctgaccgc taccacgagc aagtctctgc caagcgcaag    1320

gtggtcaaaa tgatgattgt cgtggtgtgc accttcgcca tctgctggct gcccttccac    1380

atcttcttcc tcctgcccta catcaaccca gatctctacc tgaagaagtt tatccagcag    1440

gtctacctgg ccatcatgtg gctggccatg agctccacca tgtacaaccc catcatctac    1500

tgctgcctca atgacaggtt ccgtctgggc ttcaagcatg ccttccggtg ctgccccttc    1560

atcagcgccg gcgactatga ggggctggaa atgaaatcca cccggtatct ccagacccag    1620

ggcagtgtgt acaaagtcag ccgcctggag accaccatct ccacagtggt gggggcccac    1680

gaggaggagc cagaggacgg ccccaaggcc acaccctcgt ccctggacct gacctccaac    1740

tgctcttcac gaagtgactc caagaccatg acagagagct tcagcttctc ctccaatgtg    1800

ctctcctagg ccacagggcc tttggcaggt gcagccccca ctgcctttga cctgcctccc 1860

ttcatgcatg gaaattccct tcatctggaa ccatcagaaa caccctcaca ctgggacttg 1920

caaaaagggt cagtatgggt tagggaaaac attccatcct tgagtcaaaa aatctcaatt 1980

cttccctatc tttgccaccc tcatgctgtg tgactcaaac caaatcactg aactttgctg 2040

agcctgtaaa ataaaaggtc ggaccagctt ttcccaaaag cccattcatt ccattctgga 2100

agtgactttg gctgcatgcg agtgctcatt tcaggatgaa ttctgcagca cagctgcgga 2160

cccggaagac tcattttcct ggagccccgt gttacttcaa taaagttatc tcagattagc 2220

ctcctgcagc tggaggctcc tatcacccca gcctacgctt gacagggtga acaaaagaag 2280

gcaccacata acatctaaat gaaaaattta gccctgtctt ctaagcatct gtgaaaagaa 2340

acatatgtat tccccttttt ggcatctcag tatttcagta catttataca tcatgagatt 2400

gagaacctcg ggcttccaca ttatgtcccc ggtgactgtc ctgagcagcc gacgcaagca 2460

gaatatgtcc actgatacct gctagttctc ttacagacca ggaattggga gacttgcact 2520

acatttaatg tgtagttgac cctcttttcc tacttgtaaa caaggggact gaactagata 2580

atctaagtgt tccttcgaat cttaacatcc cgtggttcaa ggattgtatg agttttttgt 2640

ttgttttaca aaaaaaaaca aaacgaagaa taaaagaata gaaaagaata ggagcagtga 2700

gtcttgtaac taatacccag ttcctggaga tgtagcaact gctaaggcca tctgtaacta 2760

tccatctcag acattctccg atttatctta aaatcctgag tacattcctt ctcatggaag 2820

gttttggctt ttgacagagc agaggacttc atgccaaggc ctgcatccat ccagctttag 2880

caggcagaat ttcatagctg cagaacactg tcagagaaga caaatgtggg ctccctgctt 2940

taaccttttg ggtattttag ggtgggggcc ctaaccttca ttcttagttt tacactagca 3000

tcgtgctcat atgtgcgaca agcaagaagg ctgcactttg cagctgcact tctgggaaga 3060

gggcatcttg catcttccct tcagactctc tgaatgtctc ctccctgctc catggctttg 3120

ccagcttcct gtctctaagg ggtagaatga ctcatcaacc ctaaaggaca gtcagtcttc 3180

caagagccat gaactgaatg ctttatatcc taatttagat ttagagtttc cagaaggtga 3240

gcatgcagtt ttgttttgtt tttttttctg tctcccaaat ctgtgttttt tccagatatg 3300

gctggaagca gaagcttcat gtaacatcca tgaatgtcct cctggtagtt tgcataatgg 3360

atgcacatgt gccgcatcca taacattaag gggagaataa tgcatggttt acagcctttg 3420

ccagccctgc tggctctaat tctaccaggg catccacagg cctgggggaa gaagaaacag 3480

tataagccag aaaacctcaa gaactacatt ctctaaagca gcatggaaag ttttaaataa 3540

actaagtgaa gccagatcat tgcagatata taaatggaag acaaaattta gaagcaacaa 3600

aagttagtgc cctaagcatt agtcatactt ccaatagaga atcttgctgt gtatggatta 3660

ctcactttgg aagaatgtaa agagctaaca tgattatgag aagtacctga gaagatggtg 3720

tcaagaagtt ggggacaccc catctatgga agagaaggtt agagttgagc tcaacgagga 3780

ttaactgagt gcctcctctg gactttgccc tgaactggga acacacagcc cctgcagctc 3840

ttgaagagcc taccttattg gccatcacta actaactcac cagtcctagt gagtctaagc 3900

tgcccagcag tcttggaggc atctgagagg acagattctc cacagaattc taaaaaccca 3960

cactcaacat gggcagtcaa gccaaagact gggacctttg gagagcctct ggaatgagag 4020

ttctctgggg tacttccaaa gggagctggc agtcagtcca ggggacctaa aggaatttgg 4080

ttgaacagta tcatctctgt gcatagtaag agggaatgtt gggtggtccg ggcagtttcc 4140

aatatggcaa agcatctgct tggacagtgc cagcaagcct tcctctgacc cagtctccaa 4200

tgtccactaa cttataaaaa tgtcatcaac tcccacatgt aagaaacacc atgatttgta 4260

ctgtgcatgg gtcacattct tattctagaa atgcatcacc ctgtgtttat ccaagtgtgt 4320

ttacttggtg taatgtccag tagtaataga atatgaaata tcaaggaacc atctttgtta 4380

cgtgacttcc aaaatgtgag atctcattgc tgtcactgtg atatttgtat tgtgtgaatc 4440

tcttcctcct cttcctcctc atgctttctc agggaggagc cctgatgtat atcatgaact 4500

cacagttcct agaccacagt aattgagggg cggtgggggg gcctttatcg gagaagctag 4560

agaacaagag tccttctcct ccttatcccc caacaggaca ctaagagaca aggactgagt 4620

ggaatcctgg agaaagggga ctcaggaact gacctcattg gcctgatttg tgaggagagg 4680

agtataagtg gagaggggcc attcctgagg tttccgtgtt ttccagcctg gtctcctgga 4740

aagaatcttt atacagaaat aaagtatgtg tttcactcaa aaaaaaaaaa aaaaa 4795

<210> 3
<211> 311
<212> PRT
<213> Homo sapiens

<400> 3

Met Asp Asn Val Leu Pro Val Asp Ser Asp Leu Ser Pro Asn Ile Ser
1               5                   10                  15

Thr Asn Thr Ser Glu Pro Asn Gln Phe Val Gln Pro Ala Trp Gln Ile
            20                  25                  30

Val Leu Trp Ala Ala Ala Tyr Thr Val Ile Val Val Thr Ser Val Val
        35                  40                  45

Gly Asn Val Val Val Met Trp Ile Ile Leu Ala His Lys Arg Met Arg
    50                  55                  60

Thr Val Thr Asn Tyr Phe Leu Val Asn Leu Ala Phe Ala Glu Ala Ser

65                  70                  75                  80

Met Ala Ala Phe Asn Thr Val Val Asn Phe Thr Tyr Ala Val His Asn
                85                  90                  95

Glu Trp Tyr Tyr Gly Leu Phe Tyr Cys Lys Phe His Asn Phe Phe Pro
            100                 105                 110

Ile Ala Ala Val Phe Ala Ser Ile Tyr Ser Met Thr Ala Val Ala Phe
        115                 120                 125

Asp Arg Tyr Met Ala Ile Ile His Pro Leu Gln Pro Arg Leu Ser Ala
    130                 135                 140

Thr Ala Thr Lys Val Val Ile Cys Val Ile Trp Val Leu Ala Leu Leu
145                 150                 155                 160

Leu Ala Phe Pro Gln Gly Tyr Tyr Ser Thr Thr Glu Thr Met Pro Ser
                165                 170                 175

Arg Val Val Cys Met Ile Glu Trp Pro Glu His Pro Asn Lys Ile Tyr
            180                 185                 190

Glu Lys Val Tyr His Ile Cys Val Thr Val Leu Ile Tyr Phe Leu Pro
        195                 200                 205

Leu Leu Val Ile Gly Tyr Ala Tyr Thr Val Val Gly Ile Thr Leu Trp
    210                 215                 220

Ala Ser Glu Ile Pro Gly Asp Ser Ser Asp Arg Tyr His Glu Gln Val
225                 230                 235                 240

Ser Ala Lys Arg Lys Val Val Lys Met Met Ile Val Val Val Cys Thr
                245                 250                 255

Phe Ala Ile Cys Trp Leu Pro Phe His Ile Phe Phe Leu Leu Pro Tyr
            260                 265                 270

Ile Asn Pro Asp Leu Tyr Leu Lys Lys Phe Ile Gln Gln Val Tyr Leu
        275                 280                 285

Ala Ile Met Trp Leu Ala Met Ser Ser Thr Met Tyr Asn Pro Ile Ile
    290                 295                 300

Tyr Cys Cys Leu Asn Asp Arg
305                 310

<210> 4
<211> 1732
<212> DNA
<213> Homo sapiens

<400> 4
agctgagcaa cccgaaccga gaggtgcccg cgaaactgca ggcggcggca gcggcagcaa 60

aagagaagga aaaatctcca gctggatacg aagctccaga atcctggcca taggctcaga 120

acttttacag gtcgcgctgc aatgggcccc cacttcgctc ctaagtcctc acgcagcaca 180

gggctttgcc tttccctgcg gaggaaggag aaataggagt tgcaggcagc agcaggtgca 240

taaatgcggg ggatctcttg cttcctagaa ctgtgaccgg tggaatttct ttcccttttt 300

cagtttaccg caagagagat gctgtctcca gacttctgaa ctcaaacgtc tcctgaagct 360

tgaaagtgga ggaattcaga gccaccgcgg gcaggcgggc agtgcatcca gaagcgttta 420

tattctgagc gccagttcag ctttcaaaaa gagtgctgcc cagaaaaagc cttccaccct 480

cctgtctggc tttagaagga ccctgagccc caggcgccag ccacaggact ctgctgcaga 540

ggggggttgt gtacagatag tagggcttta ccgcctagct tcgaaatgga taacgtcctc 600

ccggtggact cagacctctc cccaaacatc tccactaaca cctcggaacc caatcagttc 660

gtgcaaccag cctggcaaat tgtcctttgg gcagctgcct acacggtcat tgtggtgacc 720

tctgtggtgg gcaacgtggt agtgatgtgg atcatcttag cccacaaaag aatgaggaca 780

gtgacgaact attttctggt gaacctggcc ttcgcggagg cctccatggc tgcattcaat 840

acagtggtga acttcaccta tgctgtccac aacgaatggt actacggcct gttctactgc 900

aagttccaca acttctttcc catcgccgct gtcttcgcca gtatctactc catgacggct 960

gtggcctttg ataggtacat ggccatcata catcccctcc agccccggct gtcagccaca 1020

gccaccaaag tggtcatctg tgtcatctgg gtcctggctc tcctgctggc cttcccccag 1080

ggctactact caaccacaga gaccatgccc agcagagtcg tgtgcatgat cgaatggcca 1140

gagcatccga acaagattta tgagaaagtg taccacatct gtgtgactgt gctgatctac 1200

ttcctccccc tgctggtgat tggctatgca tacaccgtag tgggaatcac actatgggcc 1260

agtgagatcc ccggggactc ctctgaccgc taccacgagc aagtctctgc caagcgcaag 1320

gtggtcaaaa tgatgattgt cgtggtgtgc accttcgcca tctgctggct gcccttccac 1380

atcttcttcc tcctgcccta catcaaccca gatctctacc tgaagaagtt tatccagcag 1440

gtctacctgg ccatcatgtg gctggccatg agctccacca tgtacaaccc catcatctac 1500

tgctgcctca atgacaggtg aggatcccaa ccccatgagc tctccagggg ccacaagacc 1560

atctacatac acagtggcca agcggccatc ctaaatgagt aaacccagct gtgagacaag 1620

agggacaagt ggggactgca gctaacttat catcacacaa ctcagcctgg ctgattatca 1680

ccatccagga atgggagccc ggagtggact gattttcttt ttttcttttc ca 1732

<210>  5
<211>  407
<212>  PRT
<213>  Homo sapiens

<400>  5

Met Asp Asn Val Leu Pro Val Asp Ser Asp Leu Ser Pro Asn Ile Ser
1               5                   10                  15

Thr Asn Thr Ser Glu Pro Asn Gln Phe Val Gln Pro Ala Trp Gln Ile
            20                  25                  30

Val Leu Trp Ala Ala Ala Tyr Thr Val Ile Val Val Thr Ser Val Val
        35                  40                  45

Gly Asn Val Val Val Met Trp Ile Ile Leu Ala His Lys Arg Met Arg
    50                  55                  60

Thr Val Thr Asn Tyr Phe Leu Val Asn Leu Ala Phe Ala Glu Ala Ser
65                  70                  75                  80

Met Ala Ala Phe Asn Thr Val Val Asn Phe Thr Tyr Ala Val His Asn
                85                  90                  95

Glu Trp Tyr Tyr Gly Leu Phe Tyr Cys Lys Phe His Asn Phe Phe Pro
            100                 105                 110

Ile Ala Ala Val Phe Ala Ser Ile Tyr Ser Met Thr Ala Val Ala Phe
        115                 120                 125

Asp Arg Tyr Met Ala Ile Ile His Pro Leu Gln Pro Arg Leu Ser Ala
    130                 135                 140

Thr Ala Thr Lys Val Val Ile Cys Val Ile Trp Val Leu Ala Leu Leu
145                 150                 155                 160

Leu Ala Phe Pro Gln Gly Tyr Tyr Ser Thr Thr Glu Thr Met Pro Ser
                165                 170                 175

Arg Val Val Cys Met Ile Glu Trp Pro Glu His Pro Asn Lys Ile Tyr
            180                 185                 190

Glu Lys Val Tyr His Ile Cys Val Thr Val Leu Ile Tyr Phe Leu Pro
        195                 200                 205

Leu Leu Val Ile Gly Tyr Ala Tyr Thr Val Val Gly Ile Thr Leu Trp
    210                 215                 220

Ala Ser Glu Ile Pro Gly Asp Ser Ser Asp Arg Tyr His Glu Gln Val
225                 230                 235                 240

Ser Ala Lys Arg Lys Val Val Lys Met Met Ile Val Val Val Cys Thr
                245                 250                 255

Phe Ala Ile Cys Trp Leu Pro Phe His Ile Phe Phe Leu Leu Pro Tyr
            260                 265                 270

Ile Asn Pro Asp Leu Tyr Leu Lys Lys Phe Ile Gln Gln Val Tyr Leu
        275                 280                 285

Ala Ile Met Trp Leu Ala Met Ser Ser Thr Met Tyr Asn Pro Ile Ile
    290                 295                 300

Tyr Cys Cys Leu Asn Asp Arg Phe Arg Leu Gly Phe Lys His Ala Phe
305                 310                 315                 320

Arg Cys Cys Pro Phe Ile Ser Ala Gly Asp Tyr Glu Gly Leu Glu Met
                325                 330                 335

Lys Ser Thr Arg Tyr Leu Gln Thr Gln Gly Ser Val Tyr Lys Val Ser
            340                 345                 350

Arg Leu Glu Thr Thr Ile Ser Thr Val Val Gly Ala His Glu Glu Glu
        355                 360                 365

Pro Glu Asp Gly Pro Lys Ala Thr Pro Ser Ser Leu Asp Leu Thr Ser
    370                 375                 380

Asn Cys Ser Ser Arg Ser Asp Ser Lys Thr Met Thr Glu Ser Phe Ser
385                 390                 395                 400

Phe Ser Ser Asn Val Leu Ser
                405

<210>  6
<211>  398
<212>  PRT
<213>  Homo sapiens

<400>  6

Met Gly Thr Cys Asp Ile Val Thr Glu Ala Asn Ile Ser Ser Gly Pro
1               5                   10                  15

Glu Ser Asn Thr Thr Gly Ile Thr Ala Phe Ser Met Pro Ser Trp Gln
            20                  25                  30

Leu Ala Leu Trp Ala Thr Ala Tyr Leu Ala Leu Val Leu Val Ala Val
        35                  40                  45

Thr Gly Asn Ala Ile Val Ile Trp Ile Ile Leu Ala His Arg Arg Met
    50                  55                  60

Arg Thr Val Thr Asn Tyr Phe Ile Val Asn Leu Ala Leu Ala Asp Leu
65                  70                  75                  80

Cys Met Ala Ala Phe Asn Ala Ala Phe Asn Phe Val Tyr Ala Ser His
                85                  90                  95

Asn Ile Trp Tyr Phe Gly Arg Ala Phe Cys Tyr Phe Gln Asn Leu Phe
            100                 105                 110

Pro Ile Thr Ala Met Phe Val Ser Ile Tyr Ser Met Thr Ala Ile Ala
        115                 120                 125

Ala Asp Arg Tyr Met Ala Ile Val His Pro Phe Gln Pro Arg Leu Ser
    130                 135                 140

Ala Pro Ser Thr Lys Ala Val Ile Ala Gly Ile Trp Leu Val Ala Leu
145                 150                 155                 160

Ala Leu Ala Ser Pro Gln Cys Phe Tyr Ser Thr Val Thr Met Asp Gln
                165                 170                 175

Gly Ala Thr Lys Cys Val Val Ala Trp Pro Glu Asp Ser Gly Gly Lys
            180                 185                 190

Thr Leu Leu Leu Tyr His Leu Val Val Ile Ala Leu Ile Tyr Phe Leu
        195                 200                 205

Pro Leu Ala Val Met Phe Val Ala Tyr Ser Val Ile Gly Leu Thr Leu
    210                 215                 220

Trp Arg Arg Ala Val Pro Gly His Gln Ala His Gly Ala Asn Leu Arg
225                 230                 235                 240

His Leu Gln Ala Met Lys Lys Phe Val Lys Thr Met Val Leu Val Val
                245                 250                 255

Leu Thr Phe Ala Ile Cys Trp Leu Pro Tyr His Leu Tyr Phe Ile Leu
            260                 265                 270

Gly Ser Phe Gln Glu Asp Ile Tyr Cys His Lys Phe Ile Gln Gln Val

275                 280                 285

Tyr Leu Ala Leu Phe Trp Leu Ala Met Ser Ser Thr Met Tyr Asn Pro
    290                 295                 300

Ile Ile Tyr Cys Cys Leu Asn His Arg Phe Arg Ser Gly Phe Arg Leu
305                 310                 315                 320

Ala Phe Arg Cys Cys Pro Trp Val Thr Pro Thr Lys Glu Asp Lys Leu
                325                 330                 335

Glu Leu Thr Pro Thr Thr Ser Leu Ser Thr Arg Val Asn Arg Cys His
            340                 345                 350

Thr Lys Glu Thr Leu Phe Met Ala Gly Asp Thr Ala Pro Ser Glu Ala
        355                 360                 365

Thr Ser Gly Glu Ala Gly Arg Pro Gln Asp Gly Ser Gly Leu Trp Phe
    370                 375                 380

Gly Tyr Gly Leu Leu Ala Pro Thr Lys Thr His Val Glu Ile
385                 390                 395

<210>  7
<211>  465
<212>  PRT
<213>  Homo sapiens

<400>  7